# EUROPEAN PATENT SPECIFICATION

(11) **EP 2 734 262 B1**
(45) Date of publication and mention of the grant of the patent: **28.02.2018**
(21) Application number: 12815417.6
(22) Date of filing: 20.07.2012
(51) Int. Cl.: A61K 9/00, A61K 47/34, A61M 31/00, A61F 6/06, A61P 31/00, A61P 15/00, A61K 31/522, A61K 31/731, A61K 31/675, A61K 31/565

(54) **INTRAVAGINAL RINGS FOR DRUG DELIVERY**
INTRAVAGINALEN RINGEN ZUR WIRKSTOFFFREISETZUNG
ANNEAUX INTRAVAGINAUX POUR ADMINISTRATION DE MÉDICAMENTS

(30) Priority: 20.07.2011 US 201161509694 P; 04.06.2012 US 201261655288 P
(43) Date of publication of application: 28.05.2014
(73) Proprietor: Kiser, Patrick F., Chicago, IL 60613 (US)
(72) Inventor: SHELKE, Namdev, Salt Lake City, Utah 84108 (US); RASTOGI, Rachna, Salt Lake City, Utah 84102 (US); KISER, Patrick F., Chicago, IL 60613 (US); JOHNSON, Todd Joseph, Salt Lake City, Utah 84102 (US); CLARK, Justin Thomas, Bountiful, Utah 84010 (US)
(74) Representative: Harrison IP Limited
(86) International application number: PCT/US2012/047649
(87) International publication number: WO 2013/013172

(56) References cited:
- WO-A1-2010/138823
- WO-A2-2010/039641
- US-A- 4 493 699
- US-A1- 2006 018 951
- US-A1- 2007 043 332
- US-A1- 2011 045 076
- US-A1- 2011 045 076
- US-B1- 6 416 779
- J. ROMANO ET AL: "Safety and Availability of Dapivirine (TMC120) Delivered from an Intravaginal Ring", AIDS RESEARCH AND HUMAN RETROVIRUSES, vol. 25, no. 5, 1 May 2009 (2009-05-01), pages 483-488, XP055154696, ISSN: 0889-2229, DOI: 10.1089/aid.2008.0184
- MAKHIJA SN: 'Once daily Sustained release tablets ot Venlafaxine, a novel antidepressent' EUROPEAN JOURNAL PHARM. BIOPHARM. vol. 54, no. 1, 05 July 2002, pages 9 - 15, XP004367686

## Description

### FIELD

The present technology generally relates to intravaginal drug delivery devices. More specifically, intravaginal rings are disclosed, which are capable of providing a zero order release of loaded drugs, including hydrophilic drugs, over extended periods of time. Methods of making and using the devices are also disclosed, including prevention and/or treatment of diseases, disorders and sexually transmitted infections.

### BACKGROUND

Intravaginal drug delivery devices, including intravaginal rings (IVRs), are typically formed from biocompatible polymers and contain a drug released by diffusion through the polymer matrix. The devices may be inserted into the vaginal cavity and the drug may be absorbed by the surrounding body fluid through the vaginal tissue. In some IVR designs, the drug is uniformly dispersed or dissolved throughout the polymer matrix (monolithic system). In other designs, the drug may be confined to an inner core within the ring (reservoir system). Monolithic systems are expected to show Fickian diffusion-controlled drug release whereby the release rate decreases with time. Reservoir systems may exhibit a zero order release of loaded drugs.

To date, poly(ethylene-co-vinyl acetate), or pEVA (e.g., in NuvaRing®), and poly(dimethyl siloxane), or silicone (e.g., in Estring®, Femring® and in Population Council's progesterone-releasing ring), are currently the only polymers commercially exploited for IVRs. Compared to thermoplastics, Sn-catalyzed condensation-cured silicone is limited by a lower mechanical stiffness. Therefore, silicone IVRs are fabricated with larger cross-sectional diameters to achieve the retractive forces required for retention in the vaginal cavity, which may affect ring user acceptability. Consequently, the manufacturing costs associated with these IVRs are considerable. Moreover, pEVA and silicone are not suitable for delivery of highly water-soluble drugs and macromolecules, e.g., proteins, due to the hydrophilic nature and/or macroscopic size of such drugs. US4493699 discloses long chain alkyl and alkenyl sulfonates, sulfates and sulfoalkyl alkanoate salts, administered intravaginally for contraception.
Scientific Article "Safety and Availability of Dapivirine (TMC120) Delivered from an Intravaginal Ring" (J. Romano et al. Aids Research and Human Reteroviruses, vol. 25, no. 5, 1 May 2009, pages 483-488, XP055154696, ISSN: 0889-2229, DOI: 10.1089/aid.2008.0184) discloses a study relating to the vaginal delivery of 200 mg or 25 mg dapivirine from Intravaginal rings.
US 2011/0045076 discloses intravaginal drug delivery devices, including intravaginal rings, comprising a polyether urethane composition and a pharmaceutically effective amount of at least one vaginally administrable drug homogeneously distributed throughout the polyether urethane.

### SUMMARY

The present technology provides intravaginal drug delivery devices, in the form of intravaginal rings (IVRs), methods for making the devices, and methods of using the devices, according to claims 1, 8 and 12. Each device includes a reservoir comprising a liquid or semi-solid composition comprising one or more vaginally administrable drugs, wherein the reservoir is surrounded by a water-swellable hydrophilic elastomer. The devices are suitable for delivery of a wide variety of substances, including but not limited to hydrophilic drugs, hydrophobic drugs, and macromolecules. The one or more vaginally administrable drugs is selected from the group consisting of 1-(cyclopent-3-enylmethyl)-6-(3,5-dimethylbenzoyl)-5-ethylpyrimidine-2,4(1H,3H)-dione, 1-(cyclopentenylmethyl)-6-(3,5-dimethylbenzoyl)-5-isopropylpyrimidine-2,4(1H,3H)-dione, 1-(cyclopent-3-enylmethyl)-6-(3,5-dimethylbenzoyl)-5-isopropylpyrimidine-2,4(1H,3H)-dione, 1-(4-benzoyl-2,2-dimethylpiperazin-1-yl)-2-(3H-pyrrolo[2,3-b]pyridin-3-yl)ethane-1,2-dione, 19-norethindrone, norethisterone, levonorgestrel, norgestrel, norelgestromin, etonogestrel, gestodene, drospirenone, nomegestrol, promegestone, chlormadinone, cyproterone, medroxyprogesterone, megestrol, estrone, estriol, equilenin, equilin, zidovudine, didanosine, stavudine, lamivudine, abacavir, emtricitabine, entecavir, tenofovir, dapivirine, IQP-0528, adefovir, efavirenz, nevirapine, delavirdine, etravirine, rilpivirine, lersivirine, saquinavir, ritonavir, indinavir, nelfinavir, amprenavir, lopinavir, atazanavir, tipranavir, darunavir, elvitegravir, raltegravir, GSK-572, MK-2048, maraviroc, enfuvirtide, acyclovir, penciclovir, imiquimod, resiquimod, cisplatin, doxorubicin, paclitaxel, and combinations of two or more thereof. Because the core does not need to be heated during device fabrication, the present devices can deliver biologics, which are often susceptible to thermal degradation.

### BRIEF DESCRIPTION OF THE DRAWINGS

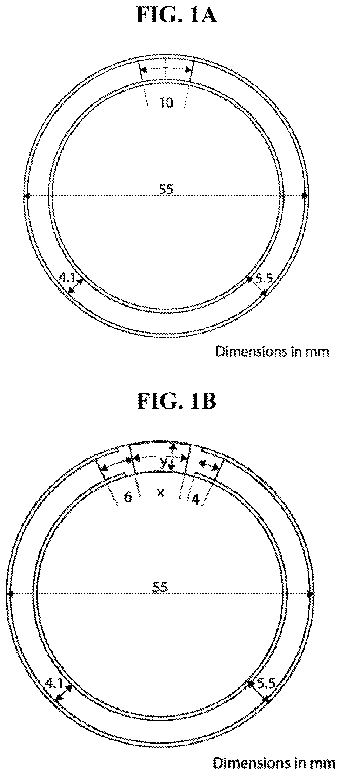

FIG. 1A shows an illustrative embodiment of a single segment reservoir IVR. FIG. 1B shows an illustrative embodiment of a two segment reservoir IVR.
FIG. 2A shows *in vitro* release of tenofovir from a reservoir IVR of the present technology. FIG. 2B shows the force required to compress exemplary IVRs of the present technology, 25% of outer diameter as described in Example 1.
FIG. 3. shows the daily release rate of LNG from the IVRs for various LNG core loadings and polymers, as described in Example 2.
FIG. 4 shows daily release rate of LNG from IVRs for various LNG tubing wall loadings, as described in reference Example 3.
FIG. 5 shows the HPLC-derived daily release rates of TFV from the IVRs described in reference Example 11.
FIG. 6 illustrate the effects of varying ratios of Tecophilic® HP-93A-100 and Tecophilic® HP-60D-60 in tubing comprising the two polymers with respect to percentage swelling.
FIG. 7 shows the 30-day average drug/active pharmaceutical ingredient (API) release from a tubular device with dry filling as described in reference Example 32.

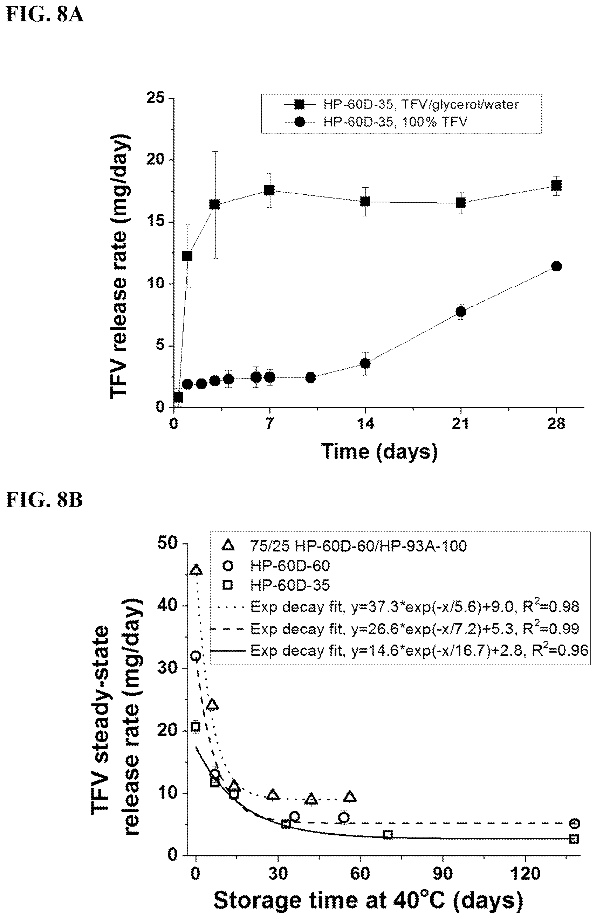

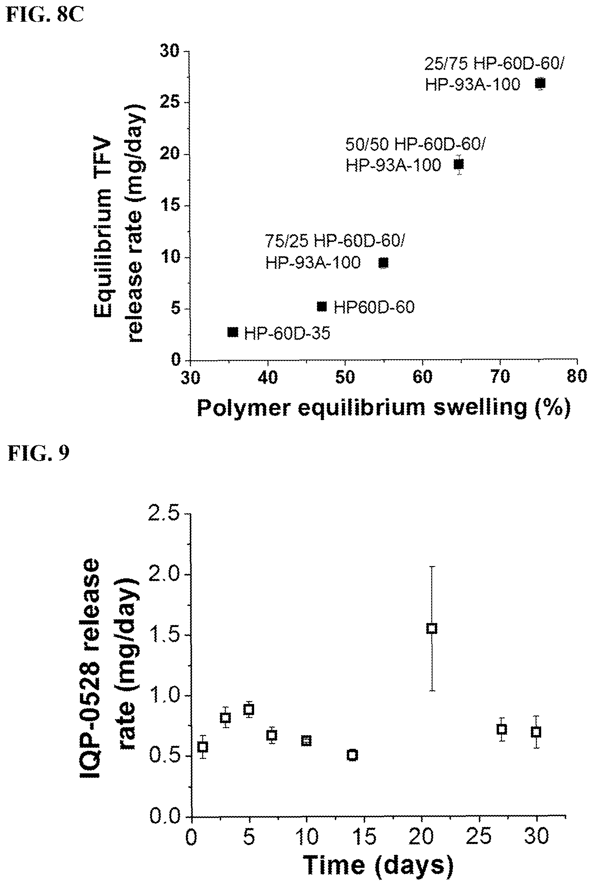

FIG. 8A is a graph showing the difference in TFV release rates (mg/day) over time with and without the osmotic agent glycerol (33 wt%) in the core of a hydrophilic tubing reservoir IVR (Tecophilic® HP-60D-35) as described in reference Example 33. FIG. 8B is a graph showing the steady-state (average from day 5 to day 14) release rate of TFV (mg/day) from Tecophilic® tubing reservoir IVRs as a function of thermal conditioning time at 40°C as described in reference Example 37. FIG. 8C shows the equilibrium (average from day 5 to day 14) release rate of TFV (mg/day) as a function of polymer equilibrium swelling ratio for various polymers and polymer blends (see reference Example 37).
FIG. 9 is a graph showing the release profile over time for a hydrophobic small molecule drug, IQP-0528 from an IVR of the present technology as described in reference Example 26.
FIG. 10 is a graph showing the release profile over time for tenofovir disoproxil fumarate (TDF) from an IVR composed of a 20 wt% swellable hydrophilic polyether urethane tubing (HydroThane™), with a reservoir containing a drug and sodium chloride mixture as described in reference Example 33.
FIG. 11 is a graph showing the release rate over time of TDF from the same IVR as in FIG. 10, after storage at elevated temperature (65°C) as described in reference Example 36.
FIG. 12A is a graph showing the release rate of elvitegravir (EVG) from the reservoir of a hydrophilic polyurethane ring. The EVG was part of a 63/5/32 wt% mixture of TFV/EVG/glycerol-water. The polyurethane was a 35 wt% swelling hydrophilic polyurethane single segment tubing with 5.5 mm cross-sectional diameter and 0.7 mm wall thickness. FIG. 12B shows the release profile of dapivirine (DPV) from the same ring with a 63/5/32 wt% mixture of TFV/DPV/glycerol-water. (See Example 38.)

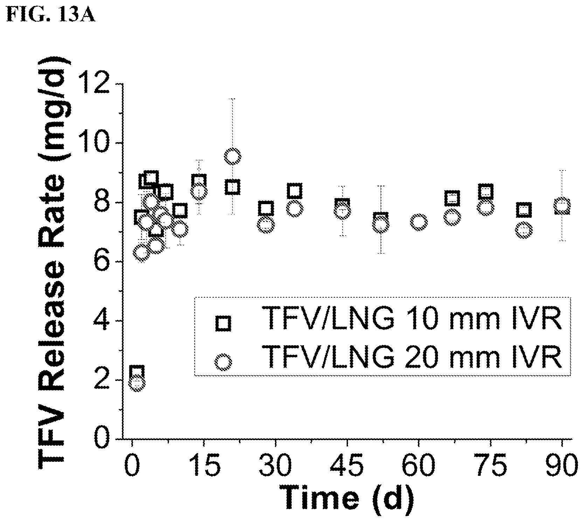

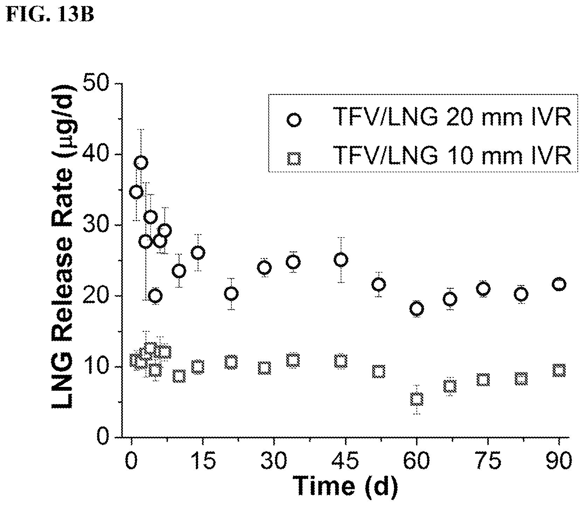

FIG. 13A is a graph showing the release rate of TFV from a hydrophilic polyurethane tubing reservoir segment of a dual-segment IVR as a function of time. The segment comprised a 35 wt% swelling hydrophilic polyurethane tube with 5.5 mm cross-sectional diameter and 0.7 mm wall thickness. FIG. 13B is a graph showing the release rate of LNG from a hydrophobic solid-core polyurethane reservoir segment, with 5.5 mm cross-sectional diameter and 0.1 mm wall thickness, of the same dual-segment IVR. The core comprised a poly(ether urethane) similar to Tecoflex EG-85A with LNG molecularly dissolved at 1.3 wt%. The outer membrane comprised a poly(ether urethane) similar to Tecoflex EG-65D and Tecoflex EG-60D. (See Example 29.)

### DETAILED DESCRIPTION

In the following detailed description, reference is made to the accompanying drawings, which form a part hereof. The embodiments described in the detailed description, drawings, and claims are illustrative. Other embodiments may be utilized, and other changes may be made.

Intravaginal rings, are provided herein. Also provided are methods for making such devices. It may be noted that such devices may find utility in methods of preventing or treating a biological condition. The devices include a reservoir of one or more of the specified vaginally administrable drugs, wherein the reservoir is surrounded by a water-swellable hydrophilic elastomer. The present devices are especially suited for, though not limited to, delivery of macromolecules and small hydrophilic molecules that are not compatible with the hydrophobic polymers widely used in current intravaginal rings due to insufficient solubility of the API/macromolecule in the hydrophobic polymer and therefore limited diffusion of hydrophilic API and macromolecules through the polymer. The present devices therefore provide a cost-effective, user-adherent/patient compliant means of providing a sustained delivery of drugs which heretofore were challenging to deliver intravaginally, including drugs that prevent the transmission of HIV or other viruses.

The intravaginal devices of the present technology include a water-swellable hydrophilic elastomer surrounding, a drug reservoir, e.g., a drug-containing core. Hydrophilic elastomers of the present technology are permeable to water and the drugs contained in the reservoir, including hydrophilic drugs. Hydrophilic elastomer swellable by water is employed in the devices. In some embodiments, the hydrophilic elastomer swells from about 20% to about 100% by weight. Examples of the amount of swelling that the hydrophilic elastomer may undergo include about 20%, about 25%, about 30%, about 35%, about 40%, about 45%, about 50%, about 55%, about 60%, about 65%, about 70%, about 75%, about 80%, about 85%, about 90%, about 95%, about 100%, and ranges between and including any two such values.

The water-swellable hydrophilic elastomer that is used in intravaginal devices of the present technology is a multiblock poly(ether urethane) comprising poly(ethylene oxide). Polyurethanes used in the present devices offer control of processing temperature, mechanical properties and drug release by modifying their components and ratios. The presence of a microphase separation leading to hard and soft domains imparts flexibility and strength to the polymer. Furthermore, poly(ether urethane)s composed of a polymeric diol and short chain diol connected by urethane linkages through diisocyanates are practically non-degradable up to three years.

A variety of medical grade poly(ether urethane)s may be used in the present devices. Such poly(ether urethane)s can be the reaction product of a polymeric diol, a short chain diol, and a diisocyanate. Diisocyanates include, but are not limited to, symmetrical molecules such as methylene-bis-cyclohexyl diisocyanates, 1,4 cyclohexyl diisocyanate, and dicyclohexyl methane diisocyanate (HMDI). Short chain diols include, but are not limited to, 1,4 butane diol or similar symmetrical diols or asymmetrical diols like 1,2 propane diol. The polymeric diols include, but are not limited to, poly(tetra methylene ether glycol) (PTMEG) and poly(ethylene glycol) (PEG). In some embodiments, the PTMEG ranges in molecular weight from about 500 to about 10,000 (e.g., 500, 1,000, 2,000, 3,000, 4,000, 5,000, 7,500, 10,000 and a range between and/or including any two such values). In others, the PEG ranges in molecular weight from about 100 to about 10,000 (e.g., 100, 250, 500, 1,000, 2,000, 3,000, 4,000, 5,000, 7,500, 10,000 and a range between and/or including any two such values). Thus, in some embodiments, the poly(ether urethane) is water-swellable and includes poly(ethylene glycol). In some embodiments, the poly(ether urethane) comprises the reaction product of dicyclohexyl methane diisocyanate, a PTMEG having a molecular weight of between about 500 and about 10,000, and 1,4 butane diol. In other embodiments, the PTMEG has a molecular weight of about 1,000 to about 2,000. In some embodiments, the number of moles of dicyclohexyl methane diisocyanate is equal to the sum of the number of moles of PTMEG and the number of moles of 1,4 butane diol and the molar ratio of 1,4 butane diol to PTMEG is between about 1 to 1 and about 1.5 to 0.5. In some embodiments, the polyurethane has an average molecular weight of about 60,000 to about 180,000 (e.g., 60,000, 80,000, 100,000, 120,000, 140,000, 150,000, 160,000, 180,000 and a range between and/or including any two such values) and a weight average molecular weight of about 120,000 to about 335,000 (e.g., 120,000, 160,000, 200,000, 240,000, 285,000, 290,000, 295,000, 300,000, 310,000, 320,000, 330,000 and a range between and/or including any two such values). These polyurethanes and their synthesis are described in detail in U.S. Pat. No. 4,523,005. These polyurethanes are also commercially available as non-swellable polyurethanes (Tecoflex® family) manufactured by Lubrizol Advanced Materials (Wickliffe, OH). Tecoflex® is a family of aliphatic polyether-based polyurethanes manufactured in several grades including but not limited to EG-80A, EG-85A, EG-93A, EG-100A, EG-60D, EG-65D, EG-68D, EG-72D. The EG-80A and EG-85A polyurethanes use a PTMEG-2000 molecular weight polyol component while the EG-93A, EG-100A, EG-60D, EG-65D, EG-68D and EG-72D polyurethanes use a PTMEG-1000 molecular weight polyol component. In addition, the ratio of the short-chain diol to the polymeric diol differs in order to vary the hardness of each grade of polyurethane. Thus, in some embodiments, the devices include a poly(ether urethane) selected from Tecoflex® EG-80A, Tecoflex® EG-85A, or Tecoflex® EG-93A. Other hydrophobic polymers that may be used include, e.g., ChronoThane™ (an aliphatic ether based polyurethane elastomer from AdvanSource Biomaterials, Wilmington, MA) T75A , T75B, T75C, and T75D, and Quadraflex® (Biomerics, Salt Lake City, UT) ALE- 75A, 80A, 85A, 90A, 93A, 95A, 55D, and 72D.

Other poly(ether urethane)s include, but are not limited to, the Tecophilic®, and Tecothane® family of polyurethanes manufactured by Lubrizol Advanced Materials. Tecophilic® is a family of aliphatic polyether-based polyurethanes, including hydrophilic water-swellable poly(ether urethane)s, manufactured by Lubrizol. Tecophilic® poly(ether urethanes) have similar composition to Tecoflex® with the addition of polyethylene glycol to impart water swellable characteristics and is available as Tecophilic® HP-60D-20, HP-60D-35, HP-60D-60, or HP-93A-100. Tecothane® is a family of aromatic polyether-based polyurethanes manufactured in several grades including TT-1074A, TT-1085A, TT-1095A, TT-1055D, TT-1065D, and TT-1075D-M. Other commercially available polyurethanes that may be used include those manufactured by DSM Biomedical (Berkeley, CA), such as PurSil®, CarboSil®, Elasthane®, BioSpan®, and Bionate®, those manufactured by Biomerics (Salt Lake City, UT), such as Quadraphilic™ ALC- and ALE- 90A-20, 90A-70, 90A-120, 55D-25, 55D-60, 55D-100, 65D-25, and 65D-60, and those manufactured by AdvanSource Biomaterials (Wilmington, MA), such as HydroThane™ AL, 80A or 93A. Any of the polyurethanes described above may be used alone or in combination to form the intravaginal devices disclosed herein.

The intravaginal devices of the present technology may further comprise additional components, including, but not limited to, other polymers or pharmaceutically compatible agents. In some embodiments, the devices further comprise PEG. In such embodiments, PEG may be present at varying amounts, including, but not limited to, amounts ranging from about 5% w/w to about 35% w/w, where w/w refers to the weight ratio of PEG to the total weight of the hydrophilic elastomer.This includes embodiments in which the amount ranges from about 5% w/w to about 15% w/w, from about 7% w/w to about 13% w/w and from about 9% w/w to about 11% w/w. In some embodiments, the hydrophilic poly(ether urethane) is Tecophilic® HP-60D-35, Tecophilic® HP-60D-60, Tecophilic® HP-93A-100, or blends thereof with varying ratios. A variety of pharmaceutically compatible agents may be used, including, but not limited to, those disclosed in U.S. Patent No. 6,951,654.

The device may have any of the structures or configurations described herein (e.g., single segment, dual segment, multi-segment) and may deliver any compatible vaginally administrable drug described herein.

The intravaginal devices of the present technology also include a reservoir. The reservoir holds (or contains) a liquid or semi-solid composition that includes one or more of the specified intravaginally administrable drugs. These compositions can, but need not, include a pharmaceutically acceptable carrier or excipient. Non-limiting examples of such carriers and excipients include glycerol, cellulose (including but not limited to hydroxyethylcellulose), castor oil, polyethylene glycol, polyoxyethylene castor oil, silicone oil, mineral oil, and poloxamer. In some embodiments, water-soluble porogens, such as salt crystals, are incorporated into the tubing wall during extrusion. Such porogens dissolve upon exposure to vaginal fluid as it permeates the device and creates pores through the tubing wall, allowing for faster drug release. In some embodiments, the drug composition includes a macromolecule and porogen. In some embodiments, the elastomer surrounding the reservoir includes the drug as well. In favorable circumstances, the drug may be mixed with the elastomer during fabrication. Alternatively, the drug migrates into the elastomer during thermal conditioning of the device after loading of the reservoir.

Mixtures of hydrophilic polymers may be blended and used to fabricate a single segment or multiple segments of IVDs of the present technology. Such mixtures allow control of the overall polymer percent swelling, polymer elastic moduli, polymer phase separation, and drug flux. Thus, devices may include mixtures of two or more hydrophilic polymers with different shore hardness and swelling indices to fabricate tubing with intermediate properties. Any of the hydrophilic poly(ether urethanes) described herein may be used including, but not limited to Tecophilic® HP-60D-20, -35 and -60, HP-93A-100 and TG-500 and -2000 and HydroThane™ 80A and 93A (5 wt% to 25 wt% swelling). In yet another example, a 3:1 mixture of Tecophilic® HP-60D-60 resin (60 wt% swelling) and Tecophilic® HP-93A-100 resin (100 wt% swelling) may be used to make tubing with intermediate swelling.

The present devices may further include a portion of the intravaginally administrable drug dispersed in the hydrophilic elastomer surrounding the reservoir. Such embodiments avoid a lag period after the device has been placed in the vagina before drug can diffuse out from the reservoir core into the vagina. In some embodiments, the amount of drug dispersed in the elastomer is about 0.05 wt% to about 10 wt% of the elastomer. Examples of the amount of drug dispersed in the elastomer include about 0.05 wt%, about 0.1 wt%, about 0.2 wt%, about 0.3 wt%, about 0.5 wt%, about 0.75 wt%, about 1 wt%, about 2 wt%, about 3 wt%, about 4 wt%, about 5 wt%, about 6 wt%, about 7 wt%, about 8 wt%, about 9 wt%, about 10 wt%, and ranges between and including any two such values.

**The one or more vaginally administrable** drugs is selected from the group consisting of 1-(cyclopent-3-enylmethyl)-6-(3,5-dimethylbenzoyl)-5-ethylpyrimidine-2,4(1H,3H)-dione, 1-(cyclopentenylmethyl)-6-(3,5-dimethylbenzoyl)-5-isopropylpyrimidine-2,4(1H,3H)-dione, 1-(cyclopent-3-enylmethyl)-6-(3,5-dimethylbenzoyl)-5-isopropylpyrimidine-2,4(1H,3H)-dione, 1-(4-benzoyl-2,2-dimethylpiperazin-1-yl)-2-(3H-pyrrolo[2,3-b]pyridin-3-yl)ethane-1,2-dione, 19-norethindrone, norethisterone, levonorgestrel, norgestrel, norelgestromin, etonogestrel, gestodene, drospirenone, nomegestrol, promegestone, chlormadinone, cyproterone, medroxyprogesterone, megestrol, estrone, estriol, equilenin, equilin, zidovudine, didanosine, stavudine, lamivudine, abacavir, emtricitabine, entecavir, tenofovir, dapivirine, IQP-0528, adefovir, efavirenz, nevirapine, delavirdine, etravirine, rilpivirine, lersivirine, saquinavir, ritonavir, indinavir, nelfinavir, amprenavir, lopinavir, atazanavir, tipranavir, darunavir, elvitegravir, raltegravir, GSK-572, MK-2048, maraviroc, enfuvirtide, acyclovir, penciclovir, imiquimod, resiquimod, cisplatin, doxorubicin, paclitaxel, and combinations of two or more thereof

The devices of the present technology include one or more vaginally administrable drugs. The devices are adapted to deliver pharmaceutically effective amounts of such drugs. By "pharmaceutically effective," it is meant an amount which is sufficient to effect the desired physiological or pharmacological change in the subject. This amount will vary depending upon such factors as the potency of the particular drug, the desired physiological or pharmacological effect, and the time span of the intended treatment. Those skilled in the pharmaceutical arts will be able to determine the pharmaceutically effective amount for any given drug in accordance with standard procedures. Thus, in some embodiments, the drug is present in the devices in an amount ranging from about 1 mg to about 2,000 mg of drug per device. Examples of amounts of drug loaded into the device include about 1 mg, about 2 mg, about 3 mg, about 4 mg, about 5 mg, about 6 mg, about 8 mg, about 10 mg, about 15 mg, about 20 mg, about 25 mg, about 30 mg, about 40 mg, about 50 mg, about 75 mg, about 100 mg, about 200 mg, about 500 mg, about 1,000 mg, about 1,500 mg, about 2,000 mg, and ranges between and including any two such values. In other embodiments, the drug is present in an amount ranging from about 0.01% w/w to about 50% w/w, where w/w refers to the weight ratio of the drug to the total weight of the device. Examples of such amounts include about 0.01%, about 0.02%, about 0.03%, about 0.05%, about 0.1%, about 0.2%, about 0. 3%, about 0.5%, about 1%, about 2%, about 5%, about 10%, about 20%, about 30%, about 40%, about 50 % w/w, and ranges between and including any two such values. In some embodiments, the drug is tenofovir and is present in an amount of about 500 mg to about 2000 mg per device. In other embodiments, the drug is levonorgestrel and is present in an amount ranging from about 1 mg to about 10 mg per device.

The intravaginal devices of the present technology are capable of providing sustained delivery of one or more vaginally administrable drugs in a substantially zero order release profile. By substantially zero order it is meant that a substantially constant amount of drug is released over a given period of time. In some embodiments, the devices exhibit a substantially zero order release profile of the drug over at least one day. In other embodiments, the devices exhibit a substantially zero order release profile of the drug over at least several days (e.g., over at least 2, 3, 4, 5, or 6 days), over at least a week, over at least one month, or over more than a month (e.g., over at least 45, 60, or 90 days). The release rate of drug from the devices of the present technology may be modified by changing the initial loading of the poly(ether urethane) matrix with drug or by modifying the components or composition of the poly(ether urethane) to make the polymer more or less hydrophobic. Additionally, changing device geometry such as surface area and tubing thickness or core excipient can be used to modify the drug release rate. In some embodiments the core loading may not affect the release rate, but will instead affect the release duration. In some embodiments, the devices exhibit release rates ranging from about 5 µg of drug per day to about 50 mg of drug per day. This release rate is expected to be sufficient to achieve the desired therapeutic concentration of drugs, including, e.g., anti-HIV agents, in the vagina to prevent sexual transmission of HIV. In other embodiments, the devices exhibit release rates of about 5µg, about 10 µg, about 25 µg, about 50µg, about 75 µg, about 100 µg, about 150 µg, about 200 µg, about 500 µg, about 750 µg, about 1 mg, about 2 mg, about 3 mg, about 4 mg, about 5 mg, about 10 mg, about 15 mg, about 20 mg, about 30 mg, about 40 mg, about 50 mg of drug per day and ranges between and including any two such values.

In some embodiments, the intravaginal device includes tubing having an interior space that that makes up the reservoir. The tubing may be a single segment or may include two or more segments, at least one of which includes the reservoir. Thus, devices of the present technology may have separate segments to deliver different drugs if so desired. At least one segment of the device includes a water-swellable hydrophilic elastomer. In others, at least one segment of the device may also be a non-swellable hydrophobic elastomer. In some embodiments, the device includes at least a segment including a water-swellable hydrophilic elastomer and a segment including a non-swellable hydrophobic elastomer. The non-swellable hydrophobic elastomer may be selected from the group consisting of hydrophobic poly(ether urethane), poly(ethylene-co-vinyl acetate), polyether amide copolymer, silicone, silicone-poly(carbonate urethane), poly(carbonate urethane), and silicone-poly(ether urethane). The two or more segments may be joined by a polymer end-cap (i.e., a solid piece of polymer such as, e.g., a plug, rod, or disk) substantially impermeable to the drug in at least one of the segments.

Multi-segment devices may include one or more tubular segments and/or one or more solid polymeric segments. Each segment may contain one or more APIs that may be different for each segment. Between each segment there may exist polymeric end caps to prevent diffusion of drug(s) into the other adjacent segments. These end caps include polymers that are substantially (i.e., completely or almost completely) impermeable to small molecule diffusion, including but not limited to ChronoThane™ T65D and Tecoflex® EG-65D polyether urethane. The length of the end cap can be adjusted so that no significant amount of API travels from one API-loaded segment to another during device storage. The end caps will be attached to the segment ends using a variety of techniques including, but not limited to injection molding or overmolding, induction welding, solvent welding, or an adhesive. Subsequently or simultaneously, segments with end caps in between may be joined together using a variety of techniques such as injection molding/overmolding, induction welding, solvent welding, or an adhesive.

In some embodiments of the present technology, the intravaginal devices further include one or more orifices connecting the reservoir to an outer surface of the device. For example, the orifices could be slits or pores. The pores may be of any suitable shape including, but not limited to circular, oval, square or rectangular. Thus, the one or more orifices may be pores with a diameter or width from about 0.1 mm to about 2 mm.

The intravaginal ring of the present technology may encompass a variety of shapes and sizes provided the device is compatible with vaginal administration to the subject and with the requirements imposed by drug delivery kinetics. In some embodiments, the intravaginal device may be strengthened by use of a mesh, braid or spring. For example, woven or braided tubing is commonly used in medical tubing applications such as catheters to give the wall sufficient strength and rigidity and prevent tube kinking as disclosed in U.S. Patent Number 5,059,375.

The device of the present technology is an intravaginal ring (IVR). The dimensions of the IVR may vary depending upon the anatomy of the subject, the amount of drug to be delivered to the patient, the time over which the drug is to be delivered, the diffusion characteristics of the drug and other manufacturing considerations. The IVR should be flexible enough to enable bending and insertion inside the vaginal cavity and rigid enough to withstand the expulsive forces of the vaginal musculature without causing abrasion to the vaginal epithelium. In some embodiments, the outer diameter of the IVRs may range, e.g., from about 45 mm to about 65 mm (including but not limited to any diameter within that range such as 50 mm, 55 mm, 60 mm and so forth). The cross-sectional diameter of the IVRs may range, e.g., from about 1.5 mm to about 10 mm (e.g., about 3, about 4, about 5, about 6, about 7, about 8, about 9, or about 10 mm and ranges between and including any two such values). The cross-sectional diameter of the reservoir core may range from about 1 mm to about 8 mm. Thus the thickness of the hydrophilic elastomer surrounding the reservoir may vary from about 0.1 mm to about 2 mm (e.g., about 0.1, about 0.2, about 0.3, about 0.4, about 0.5, about 0.6, about 0.7, about 0.8, about 0.9, about 1, about 1.1, about 1.25, about 1.5 about 1.75, or about 2 mm, and ranges between and including any two such values). The intravaginal ring may be a single segment or it may include at least two segments. Optionally, one of the segments includes a second intravaginally administrable drug different from the first. For example, in a ring with two or more segments, one segment may include any of the drugs discussed herein (e.g., an antiviral), and the second drug may be a contraceptive.

The present technology also provides methods of making the intravaginal devices disclosed herein. The methods include loading the reservoir of an intravaginal device or a precursor thereto with an intravaginally administrable drug, wherein the reservoir is surrounded at least in part by a water-swellable hydrophilic multiblock poly(ether urethane) elastomer comprising poly(ethylene oxide). Optionally, the reservoir may be loaded with a composition comprising one or more of the specified intravaginally administrable drug and a pharmaceutically acceptable carrier and/or excipient. The methods further include forming the precursor into a tube of a hydrophilic elastomer. Such a tube may be formed into an intravaginal (hollow) rod by simply sealing off each end of the tube or into an intravaginal ring by joining the two ends to each other. The device or precursor thereto may be formed by coaxial extrusion or injection molding of any of the polymers discussed herein, including but not limited to poly(ether urethane)s.

The present devices may be thermally conditioned, i.e., heated to allow the drug to diffuse into the elastomer (preventing lag times in drug release) and/or to provide increased physical/structural stability to the elastomer after fabrication. In some embodiments the device is heated from 1 to 30 days, in others, from 7 to 28 days, in still others, from 7 to 14 days. Heating must be gentle enough not to degrade the drug. In some embodiments, the device is heated to a temperature from about 30 to about 60 °C. Examples of thermal conditioning temperatures include about 30°C, about 35°C, about 40°C, about 45°C, about 50°C, about 55°C, about 60 °C, and ranges between and including any two of these values.

In some embodiments, a portion of the drug may also be dispersed in the hydrophilic elastomer itself (e.g., 0.05 wt% to 10 wt% of the total weight of the elastomer in the outer tube or 5 wt% to 70 wt% in the drug reservoir as a core). API may be incorporated into the polymer by adding API and polymer resin to a twin-screw extruder at desired feed rates using manual, gravimetric, or volumetric feeders, and extruding at a desired elevated temperature to melt and mix the polymer with API. The resultant mixture leaves the extruder as a homogenous drug-incorporated into the polymer extrudate.

In some embodiments, the ring may be formed by extruding a hydrophilic elastomer of the present technology using a crosshead die connected to an extruder known to those skilled in the art. The extruded tube is sectioned and the ends of the cut tube are joined to form a ring by a number of welding techniques including induction welding, butt welding, sonic welding and solvent welding. Two or more segments, each containing a reservoir including a different drug may be joined together in a similar fashion. Alternately, the additional segment(s) may be solid and have drug(s) dispersed throughout the polymer (e.g., homogenously) rather than contained in a reservoir. Each of these steps may be carried out under a variety of conditions, including, but not limited to those described in the Examples.

The intravaginal devices disclosed herein may be suitable for releasing the drug while the device resides in a subject's vagina. The devices may find utility in the treatment or prevention of a variety of biological conditions. As used herein, "biological condition" refers not only to diseases and disorders but conditions for which medical treatment may be desirable. Thus, the devices may find utility in the treatment or prevention of biological conditions, including, but not limited to a sexually transmitted disease, pregnancy and a post-menopausal condition. The devices may also find utility in the prevention or treatment of other biological conditions such as the bacterial, fungal, viral and/or protozoal infections disclosed in U.S. Patent No. 6,591,654. In some embodiments, the biological condition is a sexually transmitted disease, including, but not limited to HIV, HSV, HBV or HPV. The intravaginal device may be retainably positioned within the vaginal tract of the subject. The intravaginal device may be retained in place for a period of time, including, but not limited to, about one day, about several days, about one month, or more than a month.

### EXAMPLES

The present technology is further illustrated by the following examples, which should not be construed as limiting in any way.

### Example 1: Formation of Intravaginal Ring (IVR) from Poly(ether urethane)

Tecophilic® hydrophilic aliphatic extrusion grade thermoplastic polyurethane was obtained (supplied by Lubrizol Advanced Materials, Wickliffe, OH), in particular HP-60D grades with 60, 35, and 20 wt% equilibrium swelling. These polymers have a durometer (shore hardness) ranging from 41 to 43D and flexural modulus ranging from 2.76 x 10⁷ to 2.96 x 10⁷ Pa (4000 - 4300 psi). The various polymer resins were mixed with the opacifier titanium dioxide (anatase form, USP grade, Spectrum Chemicals, Gardena, CA, item number TI140, CAS number 13463-67-7) at a titanium dioxide concentration around 0.75 wt%. The different mixtures were then hot-melt extruded using a tubing crosshead (Guill Tool, West Warwick, RI) mounted on a 1.905 cm (¾ inch) 25:1 L/D single screw extruder (C.W. Brabender, South Hackensack, NJ). The tubing was extruded with heating zones ranging from 130 to 180°C and drawn down to create a product with approximately 700 µm wall thickness and 5.5 mm cross-sectional diameter (outer diameter = 5.5 mm, inner diameter = 4.1 mm). The extrudate was then cut to 155.5 mm length using a Loctite® precision o-ring cut and splice tool (Henkel, Rocky Hill, CT).

Tenofovir (TFV, manufactured by Gilead and supplied by CONRAD, Arlington, VA) was ground twice for 30 seconds each in a M20 water-cooled grinder at 20,000 rpm (IKA, Wilmington, NC). The ground TFV was then manually mixed with a spatula with the plasticizing/lubricating agent glycerol (USP grade, Spectrum Chemicals, Gardena, CA, item number G1016, CAS number 56-81-5) at a ratio of 70/30 wt%. The tubing cores were then filled with the TFV/glycerol mixture manually by packing the mixture in with a 3 mm diameter brass rod. The IVR segments were weighed before and after filling, and approximately 2.9 g of TFV/glycerol was added to each IVR, amounting to approximately 2.0 g of TFV in each IVR core. Solid core cylindrical plugs of 4.1 mm cross-sectional diameter were extruded using the same material as tubing and subsequently cut to approximately 1 cm long to serve as a mating/joining interface for the tubing ends. The plug was placed in both ends of a filled tubing segment so that the tubing ends touched each other with the plug centered roughly in-between. An RF transistorized solid state induction heating unit, HPS-20, with pneumatic air and water-cooling was used to melt and join the tubing ends and plug to create a sealed IVR (PlasticWeld Systems, Inc., Newfane, NY). The ring joint to be welded was placed in a 6 mm long hardened stainless steel split die with outer diameter of 5.5 mm. The split die closed around the joint interface and 55% power was applied to the split die for 10 seconds, followed by 10 seconds of chilled air cooling. The split die then opened and the joined ring was removed, resulting in a sealed IVR with final dimensions of 55 mm outer diameter and 5.5 mm cross-sectional diameter.

Following the split-die welding step, IVRs may adopt a non-circular conformation. To anneal IVRs into a circular conformation, IVRs are placed in a 12-cavity temperature controlled aluminum mold to shape anneal the rings in a circular shape and alleviate tube kinking. The mold is heated to 65°C for 15 minutes followed by 5 minutes at 10°C.

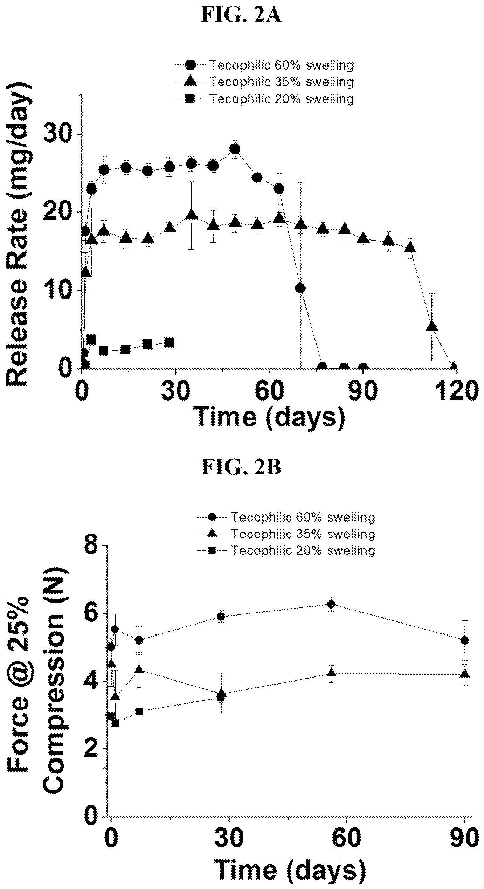

*In vitro* tenofovir (TFV) release and IVR mechanical studies were conducted to evaluate device performance. IVRs were incubated in pH 4.2 sodium acetate buffer and placed in a 37°C, 80 rpm shaker for up to 120 days. Sink conditions were maintained throughout the entire experiment, and release media was changed daily. Quantification of amount of TFV released was determined using high performance liquid chromatography (HPLC) (aqueous mobile phase gradient method on reverse phase C18 column, Agilent 1200 series HPLC). Mechanical properties of the rings over time were measured by compressing rings 25% of their initial outer diameter at a rate of 1 mm/sec using an Instron 3342 mechanical testing system (Norwood, MA) with custom machined aluminum probe. The TFV release rapidly attained steady-state, and release was dependent on polymer equilibrium swelling. The 35 wt% polymer was able to maintain steady-state release through 105 days. Also, the ring mechanical properties changed little with time, even after all drug was released. (See FIG. 2.)

TFV-containing paste is prepared similarly to that described above, with the exception that water is incorporated and the final composition is 65/33/2 TFV/glycerol/water wt%. The addition of water enhances the paste processability and working life.

### Example 2: Formation of IVR with Multiple Drugs

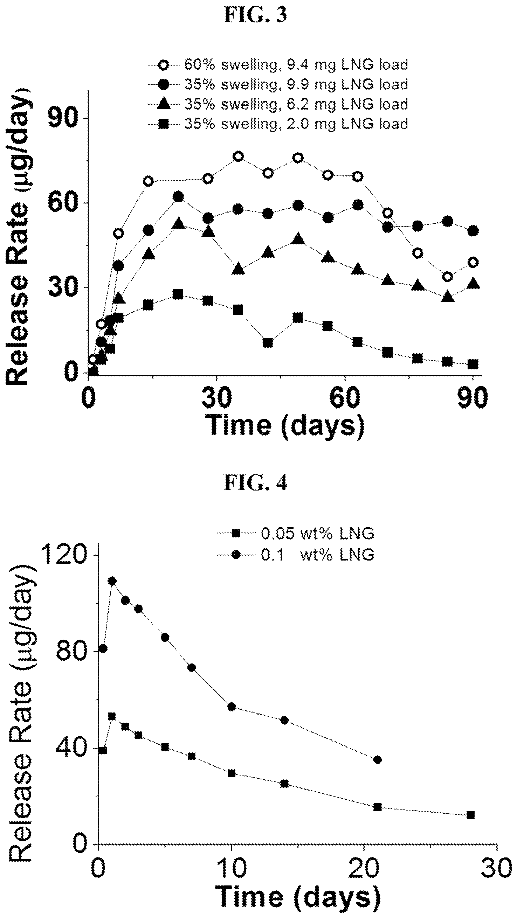

To deliver both the contraceptive Levonorgestrel (LNG, micronized, supplied by CONRAD, Arlington, VA) and TFV from a single reservoir tubing segment, polymer tubing was hot-melt extruded as described in Example 1. TFV/glycerol at a ratio of 70/30 wt% was mixed together for the core, along with a small amount (milligrams) of LNG. The tubing cores were then filled with the TFV/LNG/glycerol mixture, amounting to approximately 2.0 g of TFV and 1-10 mg LNG in each IVR core. *In vitro* LNG release studies were conducted to evaluate device performance. IVRs were incubated in sodium acetate buffer (pH 4.2) and placed in a 37°C, 80 rpm shaker for up to 90 days. Sink conditions were maintained throughout the entire experiment, and release media was changed daily. Quantification of amount of LNG released was determined using high performance liquid chromatography (HPLC) (aqueous/organic mobile phase gradient method on reverse phase C18 column, Agilent 1200 series HPLC). Steady-state LNG release was achieved, although it took approximately 21 days to achieve. Loading-dependent release was attained and 2 formulations maintained steady-state release through 90 days. (See FIG. 3.)

### Reference Example 3: Formation of IVR with Drug Dispersed in Hydrophilic Elastomer

To alleviate the roughly 21 day lag time to achieve steady-state LNG release rate as observed in Figure 2, LNG was premixed at a low concentration (0.05 - 0.2 wt%) with the hydrophilic polyurethane pellets in addition to the opacifier titanium dioxide. The mixture was then hot-melt extruded to create tubing as described in Example 1. *In vitro* release testing was performed as described in Example 2, which showed that LNG released from the tubing wall rapidly in the first couple days, common for matrix-type release. See FIG. 4. The release rate and ring mechanical properties may be modified by changing:

| | IVR O.D. | Wall thickness | X-section diameter | Polymer swelling | Polymer shore hardness | Pores/ holes | Core loading | Core excipient | Wall loading |
|---|---|---|---|---|---|---|---|---|---|
| Release rate | ✔ | ✔ | ✔ | ✔ | | ✔ | ✔ | ✔ | ✔ |
| Mechanical properties | ✔ | ✔ | ✔ | | ✔ | | | ✔ | |

### Reference Example 4: Formation of IVR with Hydrophobic Drug Dispersed in Elastomer

To deliver hydrophobic steroid contraceptives and hormone replacements which have high activity and low/precise dosing required (e.g. ethinylestradiol), the API at low wt% and polymer and titanium dioxide are premixed and extruded into tubing as described in Example 2. The tubing core composed of low wt% API is then filled as described in reference Example 3, with or without another API such as TFV or with excipient filler such as ethyl cellulose.

### Reference Example 5: Formation of IVR with Antiretroviral Drug Dispersed in Elastomer

To deliver hydrophobic antiretroviral small molecules (e.g., IQP-0528, UC-781, dapivirine, tenofovir disoproxil fumarate (TDF), elvitegravir, or GS-7340) where high delivery rates are necessary (e.g., 200 µg/day), API at a high wt% (1 wt% or greater) is compounded with polymer in a twin screw extruder and extruded into tubing per Example 1. API is then mixed at a high wt% (10 wt% or greater) with glycerol and filled as described in Example 1, with or without another API, such as TFV or with excipient filler such as ethyl cellulose.

### Example 6:

IVRs adapted to deliver other hydrophilic small molecules such as TDF, SAMT-10, acyclovir, or adefovir, are prepared as in Example 1.

### Reference Example 7: Formation of IVR for Delivery of Polymers and Proteins

For macromolecule delivery of polymers and proteins, a high wt% swelling polymer such as Tecophilic HP-93A-100 is used as the tubing polymer. Fast-dissolving porogens which do not melt at extrusion temperature and which are insoluble in the polymer but demonstrate high aqueous solubility are used to create holes and channels in the tubing wall once placed in aqueous environment and therefore increase API release rate. One such example is sodium chloride which is premixed with the polymer pellets as well as titanium dioxide and hot-melt extruded to create tubing as described in Example 1. The extruded tubing additionally may have holes created via laser cutting to allow for increased drug release if necessary. The size, shape, and number of holes are varied depending on required API release rate. The API and plasticizing/lubricating agent are subsequently mixed and filled into the tubing to create a ring as described in Example 1.

### Example 8: Formation of IVR by Coaxial Extrusion

The TFV-filled tubing may also be manufactured using an alternative coaxial extrusion method. A 12 mm twin screw extruder (C.W. Brabender) is fed TFV from a powder feeder (model K-CL-SFS-MT12 pharma twin-screw microfeeder, K-Tron, Pitman, NJ) and glycerol from a liquid feeder peristaltic pump (K-Tron). The tubing polymer is fed into a 1.905 cm (¾") single screw extruder (C.W. Brabender). The two material streams meet in a custom-made coextrusion crosshead (Guill Tool). The coextrusion crosshead inner core is liquid cooled to near room temperature to prevent the TFV/glycerol from heating. Near the end of the crosshead, the two materials meet and interface and leave the crosshead. Sections of the co-extruded product are cut and joined as described in Example 1 to create an IVR.

### Example 9: Improved IVR Manufacturing Process

In an improved manufacturing process for IVRs described in Example 1, Tecophilic® HP-60D-60 tubing was cut to 171 mm in length and one end of each tube was heat sealed closed to create a plug using a HPS-EM tip forming induction welding machine with a polytetrafluoroethylene-coated stainless steel bonding die (PlasticWeld Systems Inc., Newfane, NY). TFV/glycerol/water (65/33/2 wt%) paste was loaded into a Model 2400 Higher Pressure Filling System (Dymax, Torrington, CT) with a 150 mm stainless steel nozzle. Tubes with one end sealed were slid over the nozzle and the paste was backfilled in the tube, leaving 15 mm of the tube end free. Alternatively, 100% TFV powder was filled into tubes instead of the 65/33/2 wt% paste. Each method filled at least 1.5 g of TFV per device. Subsequently, for all filled tubes, the HPS-EM tip forming machine was used to seal the second tube end. The sealed tube ends were then welded together to form rings using the HPS-20 ring bonding induction welding system described above. Additional IVRs using other types of Tecophilic® tubing were manufactured according to this procedure.

### Example 10: Formation of Multi-Segment IVR

A multi-segment ring, wherein different APIs are delivered from different polymeric segments, is created. First, a TFV/glycerol filled tubing segment is made as described in Examples 1, 8 or 9. Another segment is fabricated using the same or different method(e.g., the additional segment may be a solid polymeric segment). Solid polymeric segments may be matrix-type (extruded or injection molded) or reservoir-type (coaxially extruded rate controlling membrane over API-loaded core). Tubular and solid polymeric reservoir-type segments may or may not contain drug within their rate controlling membrane, which would be incorporated during hot-melt extrusion or during device storage via diffusion from the drug-loaded core to the rate controlling membrane. Each segment will contain one or more API. It may be advantageous to formulate APIs in separate segments for various reasons such as independent control of each API's release rates, and chemical or physical incompatibility between the APIs or corresponding excipients.

Segments may be joined together using a variety of techniques including, but not limited to induction welding, solvent welding, or an adhesive. For example, the multiple segments are joined to a piece of high modulus polymer such as Tecoflex® EG-65D hydrophobic polyurethane (shore hardness 60D, flexural modulus 2.55 x10⁸ Pa (37,000 psi)) as this polymer is highly impermeable and has been shown to minimize diffusion of API from one segment to another. The Tecoflex® EG-65D segment(s) are then joined to the other segments to create an IVR.

Examples of APIs include, but are not limited to antimicrobial and antiviral agents, antiretroviral agents, antibacterial agents, antifungal agents, contraceptives, hormonal agents, selection progesterone receptor modulators (SPRMs, such as telapristone), and selective estrogen receptor modulators (SERMs, such as raloxifene and tamoxifen, and phytoserms from botanical source). Regarding SPRMs, the mixed agonist/antagonist activity results in selective stimulation or inhibition of progesterone-like action in divergent tissue. SERMs selectively stimulate or inhibit estrogen-like action in various tissues. SPRMs and SERMs can be delivered locally intravaginally as doses ranging between 10 µg to 100 mg. These agents can be effective therapeutics for including, but not limited to endometriosis, uterine fibroids, post-menopausal symptoms, hormone replacement therapy, anti-cancer benefits, thrombosis, and osteroporosis benefits. These agents can be delivered intravaginally over a short (1 to several days) or long (up to three months) period. Benefits of delivering these agents intravaginally include, but not limited to lower dosing, more directed therapy, and lower side-effect profile.

### Reference Example 11: Formation of IVR with Drug-Impregnated Polymeric Core

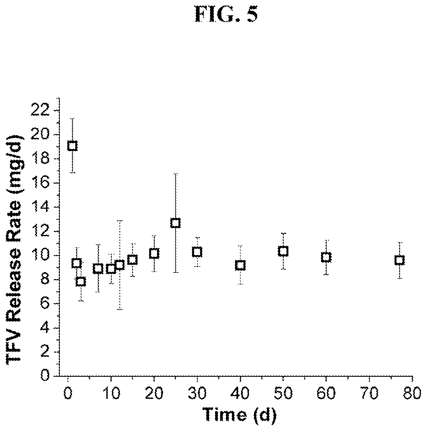

To form the core material, TFV was compounded at approximately 40% (w/w) in HPU 60, a custom-designed hydrophilic polyurethane which exhibits approximately 60% equilibrium aqueous swelling (w/w), using a twin-screw extruder to form cylindrical strands approximately 5 mm in diameter. The coating material was formed by compounding TFV at approximately 5% (w/w) into HPU 20, a custom-designed hydrophilic polyurethane which exhibits approximately 20% equilibrium aqueous swelling (w/w), using a twin-screw extruder to form cylindrical strands approximately 2 mm in diameter, which were subsequently milled in a strand pelletizer. The HPU 20 / TFV was used to jacket HPU 60 / TFV using a twin-screw extruder and crosshead die. Reservoir rods were cut to approximately 14 cm sections, of which the ends were joined using an O-ring butt-welding clamp and medical grade Tecoflex® 1-MP fast-crystallization polyurethane adhesive, (200 - 300 cps viscosity, solution of polyurethane based polymer in methyl ethyl ketone and methylene chloride) resulting in a ring with 50-60 mm outer diameter and 5-6 mm outer cross-sectional diameter. In order to ensure TFV was released in a zero-order fashion, each ring joint was then coated with a 5% HPU 20 (w/w) solution in chloroform to ensure the core material was effectively sealed in. Several of these IVRs were incubated at 37°C and in sodium acetate buffer (pH 4.2) for 77 days. Sink conditions were maintained throughout the entire experiment. High performance liquid chromatography (HPLC) analysis of release media throughout the experiment revealed that TFV was released in a zero-order fashion, following the initial burst release, for the entire experiment. (See FIG. 5.)

### Reference Example 12: Formation of IVR with Drug-Impregnated Polymeric Core

The ring described in reference Example 11 is manufactured using gravimetric (loss-in-weight) feeding and coaxial extrusion. The core and coating materials are pre-fabricated using a twin-screw extruder. For both materials, the hydrophilic polyurethane or other water-swellable polymer are fed using a gravimetric (loss-in-weight) feeder in to a twin-screw extruder while TFV is fed, down-barrel, into the same twin-extruder using a separate gravimetric loss-in-weight feeder. Material is passed through a narrow (1 - 2 mm) circular strand die at the end of the extruder barrel. The relative outputs of the two feeders are adjusted to produce the desired mass fraction of TFV in the extrudates. The ratio of equilibrium aqueous mass fractions (after swelling) of the core polymer to the coating polymer are approximately between 1.5 and 5. The mass fraction of TFV in the coating polymer is optimized to produce a burst release similar to the zero-order release profile dictated by the swelling behavior of the coating polymer and the cross-sectional geometry of the device. Both extrudates are milled into pellet form using a strand pelletizer. The core extrudate is starve-fed into a twin-screw extruder using a gravimetric feeder while the coating extrudate is flood-fed into a single-screw extruder. Both extruders are connected using a heated crosshead die in order to produce a coaxially extruded strand. The outer diameter and wall thickness of the extrudate are controlled by the relative screw speeds of the two extruders. The resulting reservoir strands are cut and joined at the ends using induction welding methods similar to those described in Examples 1 and 9, to form a ring with desired outer diameter, approximately 50 - 60 mm.

### Reference Example 13: Formation of IVR with Combination of Drugs and Drug-Impregnated Polymeric Core

A ring is prepared as described in reference Example 12 whereby a low weight fraction of levonorgestrel or other synthetic progestin is mixed with TFV prior to pre-fabrication of the core and coating extrudates in order to produce a dual protection contraceptive-microbicide IVR.

### Reference Example 14: Formation of IVR including Acyclovir and Tenofovir

A ring is prepared as described in reference Example 12, whereby a desired weight fraction of acyclovir (ACV), a hydrophilic HSV-RT inhibitor, is mixed with TFV prior to pre-fabrication of the core and coating extrudates in order to produce a microbicide IVR for the prevention or treatment of HIV and HSV infections.

### Reference Example 15: Formation of IVR including Acyclovir

A ring is prepared as described in reference Example 12, whereby TFV is replaced by ACV to be investigated for simultaneous contraception and protection against HSV infection.

### Reference Example 16: Formation of IVR with Different Drugs in Core and Wall of Ring

A ring is prepared as described in reference Example 14 whereby TFV and ACV are loaded in to separate core and coating extrudates, each coaxially extruded separately in to cylindrical strands of equal outer cross-sectional diameter. The core mass fractions of each API are generally greater than 30%. The wall thickness and relative lengths of each strand are selected to produce a desired release rate. Two coaxially extruded strands, one containing TFV and one containing ACV, are joined via two induction welds to form a ring of similar dimensions to reference Example 11.

### Reference Example 17: Formation of IVR including Tenofovir and Contraceptive

A ring is prepared as described in reference Example 16 whereby ACV is replaced by a low (<1%) mass fraction of levonorgestrel or other synthetic progestin for the same indication as the ring in reference Example 13.

### Reference Example 18: Formation of IVR including Acyclovir and Contraceptive

A ring is prepared as described in reference Example 16 whereby TFV is replaced by a low (<1%) mass fraction of levonorgestrel or other synthetic progestin for the same indication as the ring in reference Example 14.

### Reference Example 19: Formation of IVR including NNRTI

A ring is prepared as described reference Example 13 whereby the synthetic progestin is replaced by a non-nucleoside reverse transcriptase inhibitor (NNRTI) of HIV-1 such as UC781, MIV-170, MIV-150, dapivirine, efavirenz or IQP-0528, or an HIV-1 cell entry inhibitor such as maraviroc, an HIV-1 protease inhibitor such as saquinavir or ritonavir, or an HIV-1 integrase inhibitor such as darunavir or raltegravir.

### Reference Example 20: Formation of IVR including NNRTI

A ring is prepared as described in reference Example 16 whereby ACV is replaced by a low mass fraction (<10%) of a non-nucleoside reverse transcriptase inhibitor (NNRTI) of HIV-1 such as UC781, MIV-170, efavirenz or IQP-0528, or an HIV-1 cell entry inhibitor such as maraviroc, an HIV-1 protease inhibitor such as saquinavir or ritonavir, or an HIV-1 integrase inhibitor such as darunavir or raltegravir.

### Reference Example 21: Formation of IVR including NRTI

A ring is prepared as described in reference Example 13 whereby TFV is replaced by a hydrophilic nucleoside analogue reverse transcriptase inhibitor (NRTI) of HIV-1, such as emtracitibine (FTC), or an alternative NRTI such as tenofovir disoproxil fumarate (TDF).

### Reference Example 22: Formation of IVR including NRTI

A ring is prepared as described in reference Example 16 whereby TFV is replaced by a hydrophilic nucleoside analogue reverse transcriptase inhibitor (NRTI) of HIV-1 such as emtracitibine (FTC), or an alternative NRTI such as tenofovir disoproxil fumarate (TDF).

### Reference Example 23: Formation of IVR including NRTI

A ring is prepared as described in reference Example 17 whereby TFV is replaced by a hydrophilic nucleoside analogue reverse transcriptase inhibitor (NRTI) of HIV-1 such as emtracitibine (FTC), or an alternative NNRTI such as tenofovir disoproxil fumarate (TDF).

### Reference Example 24: Formation of IVR including NRTI

A ring is prepared as described in reference Example 19 whereby TFV is replaced by a hydrophilic nucleoside analogue reverse transcriptase inhibitor (NRTI) of HIV-1 such as emtracitibine (FTC), or an alternative NRTI such as tenofovir disoproxil fumarate (TDF).

### Reference Example 25: Formation of IVR including NRTI

A ring is prepared as described in reference Example 20 whereby TFV is replaced by a hydrophilic nucleoside analogue reverse transcriptase inhibitor (NRTI) of HIV-1 such as emtracitibine (FTC), or an alternative NRTI such as tenofovir disoproxil fumarate (TDF).

### Reference Example 26: Formation of IVR including Drug in Core and Wall of Ring

An IVR as described in Examples 1, 2, reference Example 3, and Example 9 is manufactured by premixing hydrophobic small molecule API (such as IQP-0528, UC-781, or levonorgestrel) with non-swellable polymer (such as Tecoflex®) and titanium dioxide. The tubing is then extruded as described above using a single screw extruder and tubing crosshead. The tubing may or may not incorporate reinforced/braided tubing or metallic springs to mechanically support the wall. The tubing is then filled with the API and excipient(s) mixture and joined to make a ring as described above. FIG. 9 shows a tubular device for hydrophobic small molecule, IQP-0528, a pyrimidinedione. The core is composed of 48 wt% drug and 52 wt% glycerol paste filled in a hydrophobic tubing (Tecoflex® EG85A) preloaded with 4.7 wt% drug. In this example, approximately 600 µg of IQP-0528 was released daily for 30 days.

### Example 27: Formation of IVR including Acyclovir

Tecophilic® hydrophilic aliphatic extrusion grade thermoplastic polyurethane (supplied by Lubrizol Advanced Materials, Wickliffe, OH) is used to deliver the antiviral agent acyclovir in a sustained and near-zero order fashion. In particular, Tecophilic® HP-60D grades with 60, 35, and 20 wt% equilibrium swelling are used. These polymers have a durometer (shore hardness) ranging from 41 to 43D and flexural modulus ranging from 2.76 x 10⁷ to 2.96 x 10⁷ Pa (4000 - 4300 psi). The various polymer resins are mixed with the opacifier titanium dioxide (anatase form, USP grade, Spectrum Chemicals, Gardena, CA, item number TI140, CAS number 13463-67-7) at a titanium dioxide concentration around 0.75 wt%. The different mixtures are then hot-melt extruded using a tubing crosshead (Guill Tool, West Warwick, RI) mounted on a 1.905 cm (¾ inch) 25:1 L/D single screw extruder (C.W. Brabender, South Hackensack, NJ). The tubing is extruded with heating zones ranging from 130 to 180°C and drawn down to create a product with approximately 700 µm wall thickness and 5.5 mm cross-sectional diameter (outer diameter = 5.5 mm, inner diameter = 4.1 mm). The extrudate is then cut to 155.5 mm length using a Loctite® precision o-ring cut and splice tool (Henkel, Rocky Hill, CT).

Micronized ACV is manually mixed with a spatula with the plasticizing/lubricating agent glycerol (USP grade, Spectrum Chemicals, Gardena, CA, item number G1016, CAS number 56-81-5) at ratios of 70/30 and 50/50 wt%. The tubing cores are then filled with the ACV/glycerol mixture by manually packing the mixture in with a 3 mm diameter brass rod. The IVR segments are weighed before and after filling, and approximately 3 g of ACV/glycerol mixture is added to each IVR. Solid core cylindrical plugs of 4.1 mm cross-sectional diameter are extruded using the same material as tubing and subsequently cut to approximately 1 cm long to serve as a mating/joining interface for the tubing ends. The plug is placed in both ends of a filled tubing segment so that the tubing ends touch each other with the plug centered roughly in-between. An RF transistorized solid state induction heating unit, HPS-20, with pneumatic air and water-cooling is used to melt and join the tubing ends and plug to create a sealed IVR (PlasticWeld Systems, Newfane, NY). The ring joint to be welded is placed in a 6 mm long hardened stainless steel split die with outer diameter of 5.5 mm. The split die closes around the joint interface and 55% power is applied to the split die for 10 seconds, followed by 10 seconds of chilled air cooling. The split die is then opened and the joined ring removed, resulting in a sealed IVR with final dimensions of 55 mm outer diameter and 5.5 mm cross-sectional diameter.

### Example 28: Formation of IVR including Contraceptive and Antiretroviral agents

Tecophilic® hydrophilic polyurethane is used to deliver both the contraceptive levonorgestrel and the antiviral agent ACV from a single segment. LNG is premixed at a low concentration (0.05 - 0.2 wt%) with the hydrophilic polyurethane pellets in addition to the opacifier titanium dioxide. The mixture is then hot-melt extruded to create tubing as described in Example 27. ACV/glycerol at a ratio of 70/30 wt% is mixed together for the core, along with a small amount (milligrams) of LNG. The tubing cores are then filled with the ACV/LNG/glycerol mixture, amounting to approximately 2.0 g of ACV and 1 - 10 mg LNG in each IVR core. The filled tube ends are then joined using the same procedure described in Example 27.

### Example 29: Formation of Dual-Segment IVR from hydrophilic and hydrophobic polymers

Example 29A. A dual-segment ring is made, wherein TFV and/or including, but not limited to, hydrophilic antiviral API (e.g., ACV, FTC) is/are delivered from a hydrophilic tubing reservoir segment and levonorgestrel and/or other contraceptive API is/are delivered from a coaxially extruded hydrophobic solid core reservoir segment. First, an TFV/glycerol filled tubing segment is made as described in Example 9. Another segment is fabricated using a coaxial extrusion setup known to those skilled in the art. Briefly, LNG and the hydrophobic polyurethane Tecoflex® EG-85A are added to a twin screw extruder at a drug loading of around 1 wt%, whereas Tecoflex® EG-65D (shore hardness 60D, flexural modulus 37,000 psi) is added to a single screw extruder. The two molten polymer feeds meet in a coaxial crosshead (Guill Tool) where the cylindrical core composed of the LNG in Tecoflex® EG-85A is coated by the Tecoflex® EG-65D with approximately 100 µm coating thickness which serves as a rate-controlling membrane. As described in Example 10, the two segments (TFV- and LNG- containing) are joined to an injection-molded plug composed of a high modulus polymer such as Tecoflex® EG-65D as this polymer is highly impermeable and has been shown to minimize diffusion of API from one segment to another. The Tecoflex® EG-65D plug segment(s) are then joined to the other segments to create an IVR. Joining methods include induction welding, solvent welding, or an adhesive.

Example 29B. In a further example, a TFV/glycerol/water (65/33/2 wt%) semi-solid paste was loaded into the hydrophilic tubing reservoir segment and LNG was dissolved in the hydrophobic solid core reservoir segment of a dual segment IVR made according to Example 29A (using similar polyurethane tubing). The resulting IVR was subjected to *in vitro* drug release testing in an aqueous buffer sink for 90 days. Zero-order release of TFV, following a brief (1 day) lag period (see Fig 13A), and near-zero-order release of LNG (see Fig 13B) were observed for 90 days.

To minimize the lag time required to reach steady-state (near-zero-order) LNG release for the hydrophobic solid reservoir segment of the dual-segment TFV/LNG IVR described herein (see FIG. 13B), the LNG-containing segments were subjected to additional thermal conditioning post-extrusion at 40°C for 14 days prior to incorporation into dual segment IVRs.

Example 29C. A dual segment ring, is made as in example 29A, where the contraceptive(s) is/are replaced by one or more polymer-soluble antiviral API (e.g. DPV, EVG, IQP-0528, GS-7340) present between 1 and 20 wt%.

### Reference Example 30: Formation of IVR for Delivery of Macromolecules

For delivery of a macromolecule such as carrageenan to prevent HPV infection, a high wt% swelling polymer such as Tecophilic® HP-93A-100 is used as the tubing polymer. The polymer tubing is extruded as described in Example 27. Fast-dissolving porogens which do not melt at extrusion temperature and which are insoluble in the polymer but demonstrate high aqueous solubility are used to create holes and channels in the tubing wall once placed in aqueous environment and therefore increase carrageenan release rate. One such example is sodium chloride which is premixed with the polymer pellets as well as titanium dioxide and hot-melt extruded to create tubing as described in Example 27. The extruded tubing additionally may have holes created via laser cutting to allow for increased drug release if necessary. The size, shape, and number of holes are varied depending on required drug release rate. Carrageenan and an excipient such as glycerol are mixed together at a ratio of approximately 70/30 wt%. Alternatively, carrageenan is compressed into pellets using a pharmaceutical pellet press. The carrageenan formulation is subsequently filled into the tubing and welded to create a ring as described in Example 27.

### Reference Example 31: Creation of tubing and devices from a mixture of polyurethane resins to obtain varying swelling

The procedure of Example 1 was followed to prepare tubing for a single or multi-segment IVD except that a blend of polymers of varying hydrophilicity was used. In a first example, a 1:1 wt/wt mixture of Tecophilic® HP 60D-35 resin (35 wt% swelling) and Tecoflex® EG-85A resin (hydrophobic) is extruded to make tubing with 21 wt% swelling.

A 3:1 wt/wt mixture of Tecophilic® HP 60D-35 resin (35 wt% swelling) and Tecoflex® EG-85A resin is extruded to make tubing with 27 wt% swelling.

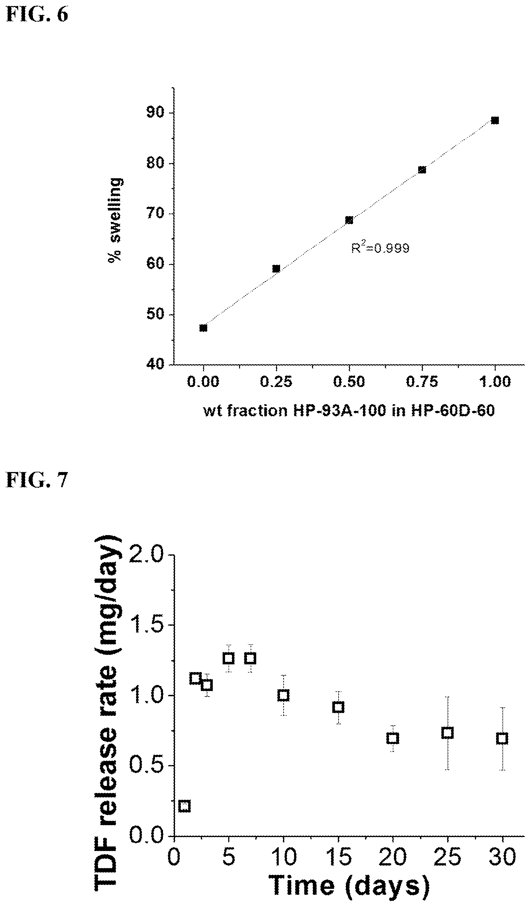

Varying ratios of Tecophilic® HP-93A-100 and Tecophilic® HP-60D-60 resins were physically mixed and extruded to make tubing whose swelling was linearly related to the ratio of the two polymer resins (see FIG. 6).

### Reference Example 32: A tubular device with solid (dry) filling

Single or multi-segment tubular devices may be filled with one or more drugs or APIs of the same or different class of compounds in the form of a powder or pellet. Devices also may be composed of one drug or API as a powder and/or pellets or two to three different drugs or APIs as powder or individual pellets depending on the desired delivery rate for each drug. Drug/API may or may not be micronized or milled or ground to a certain particle size before filling. Alternatively, the drug/API may be mixed or granulated with an excipient including, but not limited to diluents, densification, or bulking agent such as cellulose derivatives including microcrystalline cellulose, methyl cellulose, ethyl cellulose and hydroxyl propyl methyl cellulose; sugars, such as lactose and mannitol; calcium and magnesium salts, such as calcium or magnesium carbonate, di- or tribasic calcium phosphate and magnesium oxide and starch; lubricating agent and glidants to improve powder flow properties, such as magnesium, calcium or zinc stearate, talc, starch, calcium phosphate and colloidal silicon dioxide; and osmotic agents, such as salts like sodium chloride, sodium acetate, and sugars such as sucrose, mannitol, xylitol. Granulation may be done by wet or dry techniques and includes, single pot mixing, high shear mixing, spray granulation and drying, fluidized bed process, direct compression, roller compaction. Granules may be produced in various sizes, shapes, hardness, friability and possessing differing density, dissolution, and disintegration rates. The device may have one or more segments filled with immediate and delayed release formulations of same or multiple drugs/APIs, so that sufficient drug is available. Also, addition of a delayed release formulation may control the drug/API release rate from a tubing device. Delayed release formulations may be prepared by granulation or by melt extrusion followed by pelletization. FIG. 7 shows the 30-day average drug/API release from a tubular device with dry filling. A 3:1 mixture of granulated and free drug/API was filled in hydrophilic tubing for sustaining drug release over 30 days. Granules were prepared by wet granulation of drug with microcrystalline cellulose, dried and mixed in a 3:1 ratio with the API.

### Reference Example 33: A tubular device with osmotic agents or osmo-attractants to reduce lag time

Single or multi-segment tubular devices may contain an osmotic agent or osmo-attractant along with one or multiple APIs in the core. Alternatively, an osmotic agent may be incorporated in the tubing wall during extrusion or injection molding. Addition of an osmotic agent to the tubular device results in rapid entry of water into the tubing lumen and results in drug release when the device is in physiological conditions. In the absence of an osmotic agent, water entry is dependent on the rate of polymer swelling and aqueous solubility of the API resulting in delayed drug release. Higher water swelling polymers and APIs in their salt form show lower lag times. Osmotic agents include, but not limited to, low molecular weight polyvinyl pyrrolidone, polyvinyl alcohol, polyethylene glycol and polyacrylic acid. Osmotic agents also include water soluble salts, but not limited to salts of sodium and potassium, such as sodium and potassium chloride and acetate, sugars such as glucose, fructose, sucrose, trehalose, mannitol, xylitol and sorbitol and alcohols such as glycerol, ethylene glycol, propylene glycol and tetraethylene glycol

FIG. 8A shows the comparative TFV release rate profiles for a hydrophilic tubing reservoir IVR comprised of Tecophilic® HP-60D-35 filled with either a 65/33/2 wt% TFV/glycerol/water paste or 100% TFV powder. Rings with 100% TFV in the core do not achieve steady state TFV release rates by 28 days, whereas rings with the osmotic agent glycerol in the core (33 wt%) achieve steady state TFV release rates by 3 days.

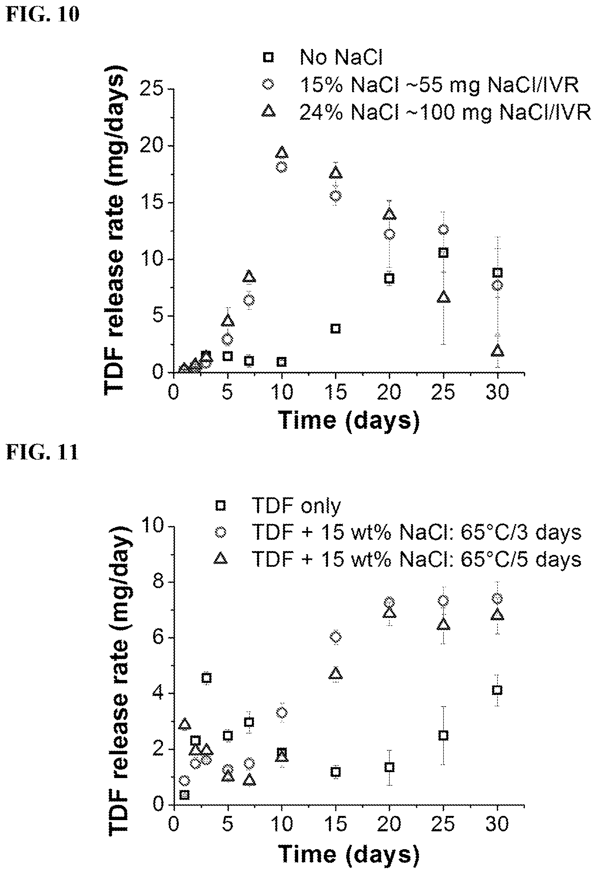

FIG. 10 shows the release profile for TDF from an IVR composed of a 20 wt% swellable hydrophilic polyether urethane tubing (HydroThane™), with core composed of a drug and sodium chloride mixture. Sodium chloride (15 wt% of total drug content) acts as an osmo-attractant and aids in decreasing the lag time for equilibrium swelling.

### Example 34: A tubular device for delivery of nucleotide analogue reverse transcriptase inhibitor, tenofovir and its prodrugs

A vaginal device of the present technology may be used to deliver a nucleotide analogue reverse transcriptase inhibitor, tenofovir or its prodrugs, tenofovir disoproxil fumarate and GS-7340, directly to the female genital tract in prophylactic doses to prevent HIV and HSV (and HPV) infections. TDF and GS-7340 are well-suited for local delivery to vaginal tissues, because of their increased hydrophobicity and therefore result in relatively higher tissue uptake in comparison to TFV. This also reduces the overall amount of the drug required for protection eventually making it possible to deliver relevant quantities up to 30 days with one time device insertion. The amount of drug delivered form the device may include 0.1 - 20 mg/day.

A device is produced by the method of Examples 1, 9 or of reference Example 26. Tubing may be composed of hydrophilic aliphatic polyether urethane or its combination with hydrophobic polyether urethane. Hydrophilic polyether urethanes may include, but not limited to Tecophilic® HP-60D-20, -35 and -60, HP-93A-100 and TG-500 and -2000 and HydroThane™ 80A and 93A (5 wt% to 25 wt% swelling) and hydrophobic polyether urethanes may include, but not limited to Tecoflex® EG-80A, 85A, 93A, 60D, 65D, 68D and 72D and ChronoThane™ T75A to 75D. Tubing dimensions are variable and wall thickness may range from 0.6 mm to 1.2 mm and diameter from 4 mm to 5.5 mm.

Tubing is filled with formulations in the reservoir. Drug may be micronized or milled to desired particle size before filling. The reservoir may be filled with drug combined with an excipient. The amount of drug filled in the core may be from 1 mg to 2000 mg. The core may contain from 0 to 80 wt% excipients. Excipients include, but not limited to diluent or bulking agent, such as cellulose derivatives, microcrystalline cellulose, methyl cellulose and ethyl cellulose; sugars, lactose, mannitol; calcium and magnesium salts, calcium or magnesium carbonate, di- or tri-basic calcium phosphate, and magnesium oxide; starch; lubricating agents and glidants to improve powder flow properties; such as magnesium, calcium or zinc stearate, talc, starch, calcium phosphate; colloidal silicon dioxide; and osmotic agents to reduce lag time, such as salts such as sodium chloride, sodium acetate and sugars like sucrose, mannitol, xylitol. Alternatively tubing may be filled with semi-solid formulation as a paste. Paste can be prepared with water, alcohols such as glycerol, ethylene glycol, propylene glycol, polyethylene glycol. Paste can also be made with oils for example, castor oil, or silicones, such as dimethicone using a high shear homogenizer and may include osmotic agents and excipients. Since prodrugs are susceptible to hydrolytic degradation, use of water or hygroscopic agents should be avoided.

Tubing may be filled with semi-solid formulation using auger or gravimetric filling techniques. Total weight of the filled material may be from 100 mg to 2000 mg. Tubes may be sealed and device may be fabricated using induction welding, solvent welding or by use of plugs held by adhesive, induction or solvent welded.

### Example 35: A tubular device for delivery of agents to promote vaginal health and treat vaginal conditions

In one embodiment, the vaginal device is made from polymer tubing with a drug filled reservoir. Tubing may be composed of hydrophilic aliphatic polyether urethane or its combination with hydrophobic polyether urethane. Hydrophilic polyether urethanes may include, but not limited to Tecophilic® HP-60D-20, -35 and -60, HP-93A-100 and TG-500 and -2000 and HydroThane™ 80A and 93A (5 wt% to 25 wt% swelling) and hydrophobic poly(ether) urethanes may include, but not limited to Tecoflex® EG-80A, 85A, 93A, 60D, 65D, 68D and 72D and ChronoThane™ T75A to 75D. Tubing dimensions are variable and wall thickness may range from 0.06 mm to 1.2 mm and diameter from 4 mm to 5.5 mm.

APIs may include agents that promote or improve vaginal health conditions. Many factors can impact vaginal health and conditions and vaginal microflora including antibiotics, menopause (or estrogen decline), oral contraceptives, spermicides, and/or diabetes. Use of tubular devices to deliver agents such as probiotics and prebiotics intravaginally will promote improved vaginal health and replace or replenish health microflora. Delivery of probiotics including, but not limited to stains of *Lactobacillus, Lactobacillus rhamnosus, Lactobacillus reuteri,* and *Lactobacillus fermentum* for maintaining healthy vaginal flora can be accomplished using such tubular intravaginal devices. Prebiotics including, but not limited to particular fructooligosaccharides, galactooligosaccharides, and lactulose also could promote vaginal health when delivered intravaginally.

### Reference Example 36: A tubing device with API incorporated in the wall at elevated temperature or extended storage

As a means of diminishing or eliminating API release lag-time, devices may be stored at elevated temperature ranging from 40°C to 70°C, depending on drug stability, for a predetermined amount of time to accelerate diffusion of the API from the tubing lumen to the tubing wall. After a predetermined time/temperature storage, the API loading in the device wall be equilibrated and the device will show minimal or no lag-time in drug release. This approach works for APIs which show some solubility in the rate-controlling polymer. Also, in certain instances this approach may negate the need for an osmotic agent. FIG. 11 shows the release profile for tenofovir disoproxil fumarate (TDF) from an IVR composed of a 20 wt% swellable hydrophilic polyether urethane tubing, HydroThane 25-93A with a reservoir filled with TDF and sodium chloride (15 wt% of TDF). The IVRs are incubated at elevated temperatures, for example 65°C for 5 days. This results in diffusion of TDF from the reservoir into the tubing wall to achieve a concentration of 5 mg/g polymer. This minimal amount of TDF in tubing wall reduced the lag time for equilibrium drug release and about 2 - 3 mg of TDF was delivered on days 1 - 3.

### Reference Example 37: Production of a thermodynamically stable IVR formulation

An inherent problem with polyurethanes is their tendency to microphase separate after thermal processing. This physical polymer rearrangement can impact API flux and device mechanical properties and creates a significant shelf life problem since the phase separation kinetics may occur on a weeks-to-years time scale. With devices created per Example 1 or Example 9, the TFV steady-state release rate has been observed to be significantly lower if devices were first stored at room temperature for several weeks prior to *in vitro* release testing. This example ensures acceleration of the polyurethane phase separation during the last manufacturing step so that the device is thermodynamically stable thereafter. TFV IVRs are prepared as described in Example 1 or Example 9, using variety of hydrophilic poly(ether) urethane (Tecophilic) tubing, including blended materials described in reference Example 31. IVRs are then placed in a vapor-flex flat barrier bag, type VF42 PET/FOIL/LLDPE (LPS Industries, Moonachie, NJ). The pouches are heat sealed using an impulse sealer Model AIE 300A. To thermally condition the polyurethane so that the devices would be stable over a two year shelf life, the pouches are placed in a 40°C oven for various time. The rings are then evaluated for TFV release kinetics using methodology described in Example 1.

When rings prepared and tested as described in Example 1 or Example 9 are thermally conditioned at 40°C prior to *in vitro* release testing, the decrease in steady-state TFV release rate (calculated by averaging the amount of TFV released on days 5 - 14) follow exponential decay kinetics, but eventually equilibration of the formulation is achieved, whereby longer storage time do not further attenuate the steady-state (or "equilibrium")TFV release rate (see FIG. 8B).

The time to equilibrium increases with polymer shore hardness/modulus (shore hardness of HP-60D-35>HP-60D-60>HP-93A-100). Bulk swelling measurements and differential scanning calorimetry of hydrated 75/25 wt% HP-60D-60/HP-93A-100 rings post-*in vitro* release testing attributed decreased TFV release rates to decreased amounts of free and partially bound water with time, which followed identical exponential decay kinetics and eventually equilibrated. The equilibrium TFV release rate (from thermally conditioned, equilibrated IVRs) increased nonlinearly with polymer equilibrium swelling (FIG. 8C), which differential scanning calorimetry identified as due to a nonlinear increase in the amount of partially bound water in hydrated polymers of higher swelling.

The 65/33/2 wt% TFV/glycerol/water formulation stored at 40°C attained equilibrium more rapidly than tubing only, which was stored for similar time and temperature, since glycerol diffused through the polymer during ring storage, acting as a plasticizer to accelerate the phase separation process.

### Example 38: Delivery of hydrophobic antiretroviral compounds at high sub-milligram/day levels

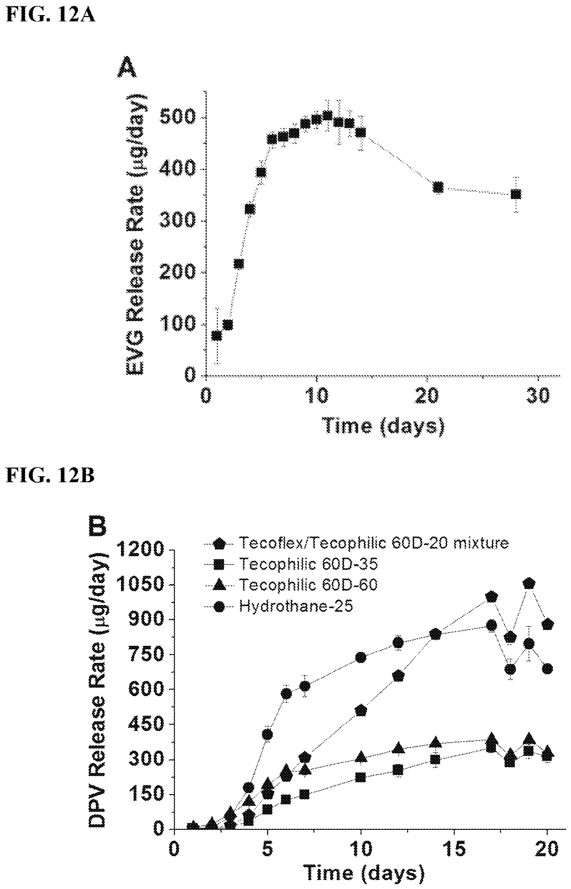

The present example demonstrates that hydrophobic compounds can be delivered from hydrophilic polyurethane tubing reservoir rings at high sub-milligram/day levels *in vitro* for over 28 days. Elvitegravir (EVG), TFV, and glycerol (63/5/32 wt% TFV/EVG/glycerol-water, where glycerol was premixed as a 33/2 wt% stock solution) was loaded into the lumen of 35 wt% swelling hydrophilic polyurethane single-segment tubing with 5.5 mm cross-sectional diameter and 0.7 mm wall thickness. EVG was released from unstored rings in a near zero order fashion at over 300 µg/day for 28 days following a several-day lag time (FIG. 12A, mean ± SD, N=5). Similarly, dapivirine (DPV) was formulated in various equilibrium swelling hydrophilic polyurethane tubing lumens (all with 5.5 mm cross-sectional diameter and 0.7 mm wall thickness) at 63/5/32 wt% TFV/DPV/glycerol-water (mean ± SD, N=5). The DPV release rates from the various hydrophilic polyurethane rings ranged from approximately 300 to 1000 µg/day (depending on polyurethane utilized), after a lag time of approximately two weeks (FIG. 12B). The highest DPV flux was achieved by polymers with the lowest swelling, indicating that DPV diffused primarily through the hydrophobic blocks of the polyurethane and thus the hydrophobic API release rate can be tailored to achieve a desired release rate. Both DPV and EVG rings described above were tested for *in vitro* release immediately after ring fabrication. It is known from above examples, as well as previously published work that amphiphilic and lipophilic compounds including DPV often are soluble in polyurethanes up to 20 wt%. Therefore, a ring delivering hydrophobic APIs, such as EVG or DPV, should show little or no lag time in releasing the drug after adequate storage time, since the API should partition into the tubing wall on ring storage. TFV release from HTPU 35 and 60% swelling rings was not noticeably affected by the presence of DPV and EVG (TFV steady-state release rates were approximately 13 and 22 mg/day, respectively).

### Example 39: Thermal Conditioning of Dual-Segment IVRs

A dual-segment IVR (Tecophilic® 75/25 HP-60D-60/HP-93A-100; Tecoflex® EG-85A coated with EG-65D) containing both TFV and LNG, was fabricated as described in Example 29A/B. To achieve all/part of combined results described in both Example 29B, where thermal conditioning was used to eliminate lag time to achieve steady-state LNG release rate, and reference Example 37, where thermal conditioning is used to provide a thermodynamically stable TFV release rate upon storage, the entire IVR (not just one of the segments) was thermally conditioned at 40°C for 14 days.

The technology illustratively described herein may suitably be practiced in the absence of any element or elements, limitation or limitations, not specifically disclosed herein. Thus, for example, the terms "comprising," "including," "containing," etc. and shall be read expansively and without limitation.

Thus, it should be understood that although the present technology has been specifically disclosed by preferred embodiments and optional features, modification, improvement and variation of the technology herein disclosed may be resorted to by those skilled in the art. The materials, methods, and examples provided here are representative of preferred embodiments, are exemplary, and are not intended as limitations on the scope of the claims.

For the purposes of this disclosure and unless otherwise specified, "a" or "an" means "one or more." All patents, applications, references and publications cited herein are incorporated by reference in their entirety to the same extent as if they were individually incorporated by reference.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into subranges as discussed above. Finally, as will be understood by one skilled in the art, a range includes each individual member.

## Claims

1. An intravaginal device comprising a reservoir surrounded by a water-swellable hydrophilic elastomer; wherein:
the water-swellable hydrophilic elastomer is a multiblock poly(ether urethane) comprising poly(ethylene oxide);
the reservoir comprises a liquid or semi-solid composition comprising one or more vaginally administrable drugs;
wherein:
the one or more vaginally administrable drugs is selected from the group consisting of 1-(cyclopent-3-enylmethyl)-6-(3,5-dimethylbenzoyl)-5-ethylpyrimidine-2,4(1H,3H)-dione, 1-(cyclopentenylmethyl)-6-(3,5-dimethylbenzoyl)-5-isopropylpyrimidine-2,4(1H,3H)-dione, 1-(cyclopent-3-enylmethyl)-6-(3,5-dimethylbenzoyl)-5-isopropylpyrimidine-2,4(1H,3H)-dione, 1-(4-benzoyl-2,2-dimethylpiperazin-1-yl)-2-(3H-pyrrolo[2,3-b]pyridin-3-yl)ethane-1,2-dione, 19-norethindrone, norethisterone, levonorgestrel, norgestrel, norelgestromin, etonogestrel, gestodene, drospirenone, nomegestrol, promegestone, chlormadinone, cyproterone, medroxyprogesterone, megestrol, estrone, estriol, equilenin, equilin, zidovudine, didanosine, stavudine, lamivudine, abacavir, emtricitabine, entecavir, tenofovir, dapivirine, IQP-0528, adefovir, efavirenz, nevirapine, delavirdine, etravirine, rilpivirine, lersivirine, saquinavir, ritonavir, indinavir, nelfinavir, amprenavir, lopinavir, atazanavir, tipranavir, darunavir, elvitegravir, raltegravir, GSK-572, MK-2048, maraviroc, enfuvirtide, acyclovir, penciclovir, imiquimod, resiquimod, cisplatin, doxorubicin, paclitaxel, and combinations of two or more thereof; and
the device is an intravaginal ring.

2. The device of claim 1, wherein the reservoir further comprises glycerol, ethylene glycol, propylene glycol, tetraethylene glycol, polyvinyl pyrrolidone, polyvinyl alcohol, polyethylene glycol, polyacrylic acid, sodium chloride, potassium chloride, sodium acetate, potassium acetate, glucose, fructose, sucrose, trehalose, mannitol, xylitol, or sorbitol.

3. The device of claim 1, wherein the reservoir further comprises glycerol.

4. The device of any one of claims 1-3, wherein the poly(ether urethane) comprises a polyurethane formed from the reaction of a diisocyanate, a polymeric diol, and a short chain diol,
optionally wherein the polyethylene glycol is present in an amount ranging from 5% w/w to 35% w/w.

5. The device of any one of claims 1-4, wherein the drug is present in an amount ranging from 1 mg to 2,000 mg of drug per device or in an amount ranging from 0.01% w/w to 50% w/w.

6. The device of anyone of claims 1-5, wherein the one or more vaginally administrable drugs is selected from the group consisting of levonorgestrel, elvitegravir, tenofovir, dapivirine, and combinations of two or more thereof.

7. The device of any one of claims 1-6, wherein the intravaginal ring comprises at least two segments, wherein one of the segments comprises a second intravaginally administerable drug different from the first, optionally wherein the second drug is a contraceptive.

8. A method of making an intravaginal device comprising loading a reservoir of an intravaginal device or a precursor thereto with a liquid or semi-solid composition comprising one or more vaginally administrable drugs, wherein the reservoir is surrounded by a water-swellable hydrophilic elastomer;
wherein:
the water-swellable hydrophilic elastomer is a multiblock poly(ether urethane) comprising poly(ethylene oxide) and the precursor is a tube of the water-swellable hydrophilic elastomer;
the one or more vaginally administrable drugs is selected from the group consisting of 1-(cyclopent-3-enylmethyl)-6-(3,5-dimethylbenzoyl)-5-ethylpyrimidine-2,4(1H,3H)-dione, 1-(cyclopentenylmethyl)-6-(3,5-dimethylbenzoyl)-5-isopropylpyrimidine-2,4(1H,3H)-dione, 1-(cyclopent-3-enylmethyl)-6-(3,5-dimethylbenzoyl)-5-isopropylpyrimidine-2,4(1H,3H)-dione, 1-(4-benzoyl-2,2-dimethylpiperazin-1-yl)-2-(3H-pyrrolo[2,3-b]pyridin-3-yl)ethane-1,2-dione, 19-norethindrone, norethisterone, levonorgestrel, norgestrel, norelgestromin, etonogestrel, gestodene, drospirenone, nomegestrol, promegestone, chlormadinone, cyproterone, medroxyprogesterone, megestrol, estrone, estriol, equilenin, equilin, zidovudine, didanosine, stavudine, lamivudine, abacavir, emtricitabine, entecavir, tenofovir, dapivirine, IQP-0528, adefovir, efavirenz, nevirapine, delavirdine, etravirine, rilpivirine, lersivirine, saquinavir, ritonavir, indinavir, nelfinavir, amprenavir, lopinavir, atazanavir, tipranavir, darunavir, elvitegravir, raltegravir, GSK-572, MK-2048, maraviroc, enfuvirtide, acyclovir, penciclovir, imiquimod, resiquimod, cisplatin, doxorubicin, paclitaxel, and combinations of two or more thereof; and
the precursor is formed into the shape of an intravaginal ring and the device is an intravaginal ring;
optionally wherein, the device is thermally conditioned from 30 °C to 60 °C and/or the device is formed by coaxial extrusion or injection molding.

9. The method of claim 8, wherein the one or more vaginally administrable drugs is selected from the group consisting of levonorgestrel, elvitegravir, tenofovir, dapivirine, and combinations of two or more thereof

10. The method of claim 8 or 9, wherein the reservoir further comprises glycerol, ethylene glycol, propylene glycol, tetraethylene glycol, polyvinyl pyrrolidone, polyvinyl alcohol, polyethylene glycol, polyacrylic acid, sodium chloride, potassium chloride, sodium acetate, potassium acetate, glucose, fructose, sucrose, trehalose, mannitol, xylitol, or sorbitol.

11. The method of claim 8 or 9, wherein the reservoir further comprises glycerol.

12. An intravaginal device of any one of claims 1-7 for use in administering the one or more vaginally administerable drug to a subject in need thereof.

## Patentansprüche

1. Intravaginale Vorrichtung umfassend einen Speicher umgeben von einem wasserquellbaren hydrophilen Elastomer;
wobei:
das wasserquellbare hydrophile Elastomer ein Multiblock-Poly(etherurethan) umfassend Poly(ethylenoxid);
der Speicher eine flüssige oder halbfeste Zusammensetzung umfasst, umfassend ein oder mehrere vaginal verabreichbare Arzneimittel; wobei:
das eine oder die mehreren vaginal verabreichbaren Arzneimittel ausgewählt sind aus der Gruppe bestehend aus 1-(Cyclopent-3-enylmethyl)-6-(3,5-dimethylbenzoyl)-5-ethylpyrimidin-2,4(1H,3H)-dion, 1-(Cyclopentenylmethyl)-6-(3,5-dimethylbenzoyl)-5-isopropylpyrimidin-2,4(1H,3H)-dion, 1-(Cyclopent-3-enylmethyl)-6-(3,5-dimethylbenzoyl)-5-isopropylpyrimidin-2,4(1H,3H)-dion, 1-(4-Benzoyl-2,2-dimethylpiperazin-1-yl)-2-(3H-pyrrolo[2,3-b]pyridin-3-yl)ethan-1,2-dion, 19-Norethindron, Norethisteron, Levonorgestrel, Norgestrel, Norelgestromin, Etonogestrel, Gestoden, Drospirenon, Nomegestrol, Promegeston, Chlormadinon, Cyproteron, Medroxyprogesteron, Megestrol, Estron, Estriol, Equilenin, Equilin, Zidovudin, Didanosin, Stavudin, Lamivudin, Abacavir, Emtricitabin, Entecavir, Tenofovir, Dapivirin, IQP-0528, Adefovir, Efavirenz, Nevirapin, Delavirdin, Etravirin, Rilpivirin, Lersivirin, Saquinavir, Ritonavir, Indinavir, Nelfmavir, Amprenavir, Lopinavir, Atazanavir, Tipranavir, Darunavir, Elvitegravir, Raltegravir, GSK-572, MK-2048, Maraviroc, Enfuvirtid, Acyclovir, Penciclovir, Imiquimod, Resiquimod, Cisplatin, Doxorubicin, Paclitaxel und Kombinationen von zwei oder mehreren davon; und die Vorrichtung ein intravaginaler Ring ist.

2. The Vorrichtung nach Anspruch 1, wobei der Speicher ferner Glyzerin, Ethylenglykol, Propylenglykol, Tetraethylenglykol, Polyvinylpyrrolidon, Polyvinylalkohol, Polyethylenglykol, Polyacrylsäure, Natriumchlorid, Kaliumchlorid, Natriumacetat, Kaliumacetat, Glukose, Fruktose, Saccharose, Trehalose, Mannitol, Xylitol oder Sorbitol umfasst.

3. Vorrichtung nach Anspruch 1, wobei der Speicher ferner Glyzerin umfasst.

4. Vorrichtung nach einem der Ansprüche 1-3, wobei das Poly(etherurethan) ein Polyurethan umfasst, das aus der Reaktion eines Diisocyanats, eines polymeren Diols und eines kurzkettigen Diols gebildet ist,
optional wobei das Polyethylenglykol in einer Menge im Bereich von 5 Gew.-% bis 35 Gew.-% vorhanden ist.

5. Vorrichtung nach einem der Ansprüche 1-4, wobei das Arzneimittel in einer Menge im Bereich von 1 mg bis 2000 mg Arzneimittel pro Vorrichtung oder in einer Menge im Bereich von 0,01 Gew.-% bis 50 Gew.-% vorhanden ist.

6. Vorrichtung nach einem der Ansprüche 1-5, wobei das eine oder die mehreren vaginal verabreichbaren Arzneimittel ausgewählt sind aus der Gruppe bestehend aus Levonorgestrel, Elvitegravir, Tenofovir, Dapivirin und Kombinationen aus zwei oder mehreren davon.

7. Vorrichtung nach einem der Ansprüche 1-6, wobei der intravaginale Ring mindestens zwei Segmente umfasst, wobei eines der Segmente ein zweites intravaginal verabreichbares Arzneimittel umfasst, das sich vom ersten unterscheidet, optional wobei das zweite Arzneimittel ein Antikonzeptivum ist.

8. Verfahren zum Herstellen einer intravaginalen Vorrichtung, umfassend ein Laden eines Speichers einer intravaginalen Vorrichtung oder eines Vorläufers davon mit einer flüssigen oder halbfesten Zusammensetzung umfassend ein oder mehrere varabreichbare Arzneimittel, wobei der Speicher von einem wasserquellbaren hydrophilen Elastomer umgeben ist; wobei:
das wasserquellbare hydrophile Elastomer ein Multiblock-Poly(etherurethan) umfassend Poly(ethylenoxid) ist, und der Vorläufer eine Röhre des wasserquellbaren hydrophilen Elastomers ist;
das eine oder die mehreren vaginal verabreichbaren Arzneimittel ausgewählt sind aus der Gruppe bestehend aus 1-(Cyclopent-3-enylmethyl)-6-(3,5-dimethylbenzoyl)-5-ethylpyrimidin-2,4(1H,3H)-dion, 1-(Cyclopentenylmethyl)-6-(3,5-dimethylbenzoyl)-5-isopropylpyrimidin-2,4(1H,3H)-dion, 1-(Cyclopent-3-enylmethyl)-6-(3,5-dimethylbenzoyl)-5-isopropylpyrimidin-2,4(1H,3H)-dion, 1-(4-Benzoyl-2,2-dimethylpiperazin-1-yl)-2-(3H-pyrrolo[2,3-b]pyridin-3-yl)ethan-1,2-dion, 19-Norethindron, Norethisteron, Levonorgestrel, Norgestrel, Norelgestromin, Etonogestrel, Gestoden, Drospirenon, Nomegestrol, Promegeston, Chlormadinon, Cyproteron, Medroxyprogesteron, Megestrol, Estron, Estriol, Equilenin, Equilin, Zidovudin, Didanosin, Stavudin, Lamivudin, Abacavir, Emtricitabin, Entecavir, Tenofovir, Dapivirin, IQP-0528, Adefovir, Efavirenz, Nevirapin, Delavirdin, Etravirin, Rilpivirin, Lersivirin, Saquinavir, Ritonavir, Indinavir, Nelfmavir, Amprenavir, Lopinavir, Atazanavir, Tipranavir, Darunavir, Elvitegravir, Raltegravir, GSK-572, MK-2048, Maraviroc, Enfuvirtid, Acyclovir, Penciclovir, Imiquimod, Resiquimod, Cisplatin, Doxorubicin, Paclitaxel und Kombinationen von zwei oder mehreren davon; und die Vorrichtung ein intravaginaler Ring ist.
optional wobei die Vorrichtung thermisch von 30 °C bis 60 °C konditioniert ist, und/oder die Vorrichtung durch koaxiales Strangpressen oder Spritzgießen gebildet ist.

9. Verfahren nach Anspruch 8, wobei das eine oder die mehreren vaginal verabreichbaren Arzneimittel ausgewählt sind aus der Gruppe bestehend aus Levonorgestrel, Elvitegravir, Tenofovir, Dapivirin und Kombinationen aus zwei oder mehreren davon.

10. Verfahren nach Anspruch 8 oder 9, wobei der Speicher ferner Glyzerin, Ethylenglykol, Propylenglykol, Tetraethylenglykol, Polyvinylpyrrolidon, Polyvinylalkohol, Polyethylenglykol, Polyacrylsäure, Natriumchlorid, Kaliumchlorid, Natriumacetat, Kaliumacetat, Glukose, Fruktose, Saccharose, Trehalose, Mannitol, Xylitol oder Sorbitol umfasst.

11. Verfahren nach Anspruch 8 oder 9, wobei der Speicher ferner Glyzerin umfasst.

12. Intravaginale Vorrichtung nach einem der Ansprüche 1-7 zur Verwendung beim Verabreichen des einen oder der mehreren vaginal verabreichbaren Arzneimittel an ein Subjekt, das sie benötigt.

## Revendications

1. Dispositif intravaginal comprenant un réservoir entouré d'un élastomère hydrophile pouvant gonfler dans l'eau ;
dans lequel :
l'élastomère hydrophile pouvant gonfler dans l'eau est un poly(éther-uréthane) multiséquencé comprenant de l'oxyde de polyéthylène ;
le réservoir comprend une composition liquide ou semi-solide comprenant un ou plusieurs médicaments administrables par voie vaginale ;
lesdits un ou plusieurs médicaments administrables par voie vaginale étant choisis dans le groupe constitué par la 1-(cyclopent-3-énylméthyl)-6-(3,5-diméthylbenzoyl)-5-éthylpyrimidine-2,4(1H,3H)-dione, la 1-(cyclopenténylméthyl)-6-(3,5-diméthylbenzoyl)-5-isopropylpyrimidine-2,4(1H,3H)-dione, la 1-(cyclopent-3-énylméthyl)-6-(3,5-diméthylbenzoyl)-5-isopropylpyrimidine-2,4(1H,3H)-dione, la 1-(4-benzoyl-2,2-diméthylpipérazin-1-yl)-2-(3H- pyrrolo[2,3-b]pyridin-3-yl)éthane-1,2-dione, la 19-noréthindrone, la noréthistérone, le lévonorgestrel, le norgestrel, la norelgestromine, l'étonogestrel, le gestodène, la drospirénone, le nomégestrol, la promégestone, la chlormadinone, la cyprotérone, la médroxyprogestérone, le mégestrol, l'estrone, l'estriol, l'équilénine, l'équiline, la zidovudine, la didanosine, la stavudine, la lamivudine, l'abacavir, l'emtricitabine, l'entécavir, le ténofovir, la dapivirine, l'IQP-0528, l'adéfovir, l'éfavirenz, la névirapine, la délavirdine, l'étravirine, la rilpivirine, la lersivirine, le saquinavir, le ritonavir, l'indinavir, le nelfinavir, l'amprénavir, le lopinavir, l'atazanavir, le tipranavir, le darunavir, l'elvitégravir, le raltégravir, le GSK-572, le MK-2048, le maraviroc, l'enfuvirtide, l'acyclovir, le penciclovir, l'imiquimod, le résiquimod, le cisplatine, la doxorubicine, le paclitaxel, et des combinaisons de deux de ceux-ci ou plus ; et
ledit dispositif étant un anneau intravaginal.

2. Dispositif selon la revendication 1, ledit réservoir comprenant en outre du glycérol, de l'éthylène glycol, du propylène glycol, du tétraéthylène glycol, de la polyvinylpyrrolidone, de l'alcool polyvinylique, du polyéthylène glycol, de l'acide polyacrylique, du chlorure de sodium, du chlorure de potassium, de l'acétate de sodium, de l'acétate de potassium, du glucose, du fructose, du saccharose, du tréhalose, du mannitol, du xylitol, ou du sorbitol.

3. Dispositif selon la revendication 1, ledit réservoir comprenant en outre du glycérol.

4. Dispositif selon l'une quelconque des revendications 1 à 3, ledit poly(éther-uréthane) comprenant un polyuréthane formé à partir d'un diisocyanate, d'un diol polymérique, et d'un diol à chaîne courte,
éventuellement ledit polyéthylène glycol étant présent en une quantité allant de 5 % en poids/poids à 35 % en poids/poids.

5. Dispositif selon l'une quelconque des revendications 1 à 4, ledit médicament étant présent en une quantité allant de 1 mg à 2000 mg de médicament par dispositif ou en une quantité allant de 0,01 % en poids/poids à 50 % en poids/poids.

6. Dispositif selon l'une quelconque des revendications 1 à 5, lesdits un ou plusieurs médicaments administrables par voie vaginale étant choisis dans le groupe constitué par le lévonorgestrel, l'elvitégravir, le ténofovir, la dapivirine, et des combinaisons de deux de ceux-ci ou plus.

7. Dispositif selon l'une quelconque des revendications 1 à 6, ledit anneau intravaginal comprenant au moins deux segments, l'un des segments comprenant un second médicament administrable par voie vaginale différent du premier, éventuellement ledit second médicament étant un contraceptif.

8. Procédé de fabrication d'un dispositif intravaginal comprenant le chargement d'un réservoir d'un dispositif intravaginal ou d'un précurseur de celui-ci avec une composition liquide ou semi-solide comprenant un ou plusieurs médicaments administrables par voie vaginale, ledit réservoir étant entouré d'un élastomère hydrophile pouvant gonfler dans l'eau ;
dans lequel :
l'élastomère hydrophile pouvant gonfler dans l'eau est un poly(éther-uréthane) multiséquencé comprenant de l'oxyde de polyéthylène et le précurseur est un tube de l'élastomère hydrophile pouvant gonfler dans l'eau ;
lesdits un ou plusieurs médicaments administrables par voie vaginale sont choisis dans le groupe constitué par la 1-(cyclopent-3-énylméthyl)-6-(3,5-diméthylbenzoyl)-5-éthylpyrimidine-2,4(1H,3H)-dione, la 1-(cyclopenténylméthyl)-6-(3,5-diméthylbenzoyl)-5-isopropylpyrimidine-2,4(1H,3H)-dione, la 1-(cyclopent-3-énylméthyl)-6-(3,5-diméthylbenzoyl)-5-isopropylpyrimidine-2,4(1H,3H)-dione, la 1-(4-benzoyl-2,2-diméthylpipérazin-1-yl)-2-(3H- pyrrolo[2,3-b]pyridin-3-yl)éthane-1,2-dione, la 19-noréthindrone, la noréthistérone, le lévonorgestrel, le norgestrel, la norelgestromine, l'étonogestrel, le gestodène, la drospirénone, le nomégestrol, la promégestone, la chlormadinone, la cyprotérone, la médroxyprogestérone, le mégestrol, l'estrone, l'estriol, l'équilénine, l'équiline, la zidovudine, la didanosine, la stavudine, la lamivudine, l'abacavir, l'emtricitabine, l'entécavir, le ténofovir, la dapivirine, l'IQP-0528, l'adéfovir, l'éfavirenz, la névirapine, la délavirdine, l'étravirine, la rilpivirine, la lersivirine, le saquinavir, le ritonavir, l'indinavir, le nelfinavir, l'amprénavir, le lopinavir, l'atazanavir, le tipranavir, le darunavir, l'elvitégravir, le raltégravir, le GSK-572, le MK-2048, le maraviroc, l'enfuvirtide, l'acyclovir, le penciclovir, l'imiquimod, le résiquimod, le cisplatine, la doxorubicine, le paclitaxel, et des combinaisons de deux de ceux-ci ou plus ; et le précurseur est formé sous la forme d'un anneau intravaginal et le dispositif est un anneau intravaginal ;
éventuellement ledit dispositif étant thermiquement conditionné de 30°C à 60°C et/ou ledit dispositif étant formé par extrusion coaxiale ou moulage par injection.

9. Procédé selon la revendication 8, lesdits un ou plusieurs médicaments administrables par voie vaginale étant choisis dans le groupe constitué par le lévonorgestrel, l'elvitégravir, le ténofovir, la dapivirine, et des combinaisons de deux de ceux-ci ou plus.

10. Procédé selon la revendication 8 ou 9, ledit réservoir comprenant en outre du glycérol, de l'éthylène glycol, du propylène glycol, du tétraéthylène glycol, de la polyvinylpyrrolidone, de l'alcool polyvinylique, du polyéthylène glycol, de l'acide polyacrylique, du chlorure de sodium, du chlorure de potassium, de l'acétate de sodium, de l'acétate de potassium, du glucose, du fructose, du saccharose, du tréhalose, du mannitol, du xylitol, ou du sorbitol.

11. Procédé selon la revendication 8 ou 9, ledit réservoir comprenant en outre du glycérol.

12. Dispositif intravaginal selon l'une quelconque des revendications 1 à 7 pour utilisation dans l'administration d'un ou plusieurs médicaments administrables par voie vaginale à un sujet en ayant besoin.
